# EUROPEAN PATENT APPLICATION

(11) **EP 4 438 620 A1**
(43) Date of publication of application: **02.10.2024**
(21) Application number: 23165585.3
(22) Date of filing: 30.03.2023
(51) Int. Cl.: C07K 14/47, C12N 15/01, C12N 15/09, C12N 15/90, C12N 5/071

(54) **CHO CELLS WITH OPTIMIZED HOST CELL PROTEIN PROFILE**

(71) Applicant: Sartorius Stedim Cellca GmbH, 89081 Ulm (DE)
(72) Inventor: Zehe, Christoph, 89081 Ulm (DE); Leroux, Ann-Cathrin, 89081 Ulm (DE); Marzluf, Jannis, 89081 Ulm (DE); Reif, Oscar-Werner, 89081 Ulm (DE)
(74) Representative: Eisenführ Speiser

(57) **Abstract**

The present invention relates to a CHO cell with a modified host cell protein (HCP) profile characterized by a reduced load of difficult-to-remove HCPs (drHCP) and/or abundant core HCPs (acHCP). The present invention also relates to a method for the production of at least one modified CHO cell according to the present invention. The present invention also relates to a method for the production of at least one recombinant molecule, preferably at least one recombinant protein, of interest and the use thereof for the production of at least one pharmaceutical drug. The present invention also relates to a kit providing all suitable reagents to perform the methods disclosed.

## Description

### Technical Field

The present invention relates to a CHO cell with a modified host cell protein (HCP) profile characterized by a reduced load of difficult-to-remove HCPs (drHCP) and/or abundant core HCPs (acHCP). The present invention also relates to a method forthe production of at least one modified CHO cell according to the present invention. The present invention also relates to a method for the production of at least one recombinant molecule, preferably at least one recombinant protein, of interest and the use thereof for the production of at least one pharmaceutical drug. The present invention also relates to a kit.

### Background of the Invention

The manufacturing of biotherapeutic agents, including antibodies and other therapeutic drugs, represents an important pillar of drug manufacturing. Typically, biotherapeutic agents, which are also referred to as a biopharmaceuticals or biologics, are produced from cells (bacterial, yeast, plant or mammalian), which are referred to as host cells or host cell lines, using recombinant DNA technology. Chinese Hamster Ovary (CHO) cells are the workhorse forthe manufacture of biopharmaceuticals. Indeed, CHO cells for manufacturing biopharmaceuticals account for over 70% of recombinant biopharmaceutical proteins, most of them being monoclonal antibodies (mAbs) and 35.5% of the total cumulative (1982-2014) biopharmaceutical product approvals (Lalonde 2017 (http://dx.doi.org/10.1016/j.jbiotec.2017.04.028), Walsh, 2014, Nat Biotechnol. 2014; 32:992-1000). The dominant use of CHO as host cells is due to their capacity to correctly fold and post-translationally modify recombinant proteins and thus make them compatible for humans (Jaypal et al., 2007, Chemical Engineering Progress 103(10):40-47). In addition, CHO cells can produce human-like glycosylation patterns when producing proteins, such as antibodies and other glycosylated molecules, which advantageously avoids undesired immune responses. Further, CHO cells are fast-growing cells capable of producing large amounts of recombinant proteins via their secretory pathway.

During the highly controlled production and sequential purification process, not only high yields of functional biopharmaceuticals are desired, but also ultimate purity of the administered composition. Host cell proteins (HCPs) are process-related protein impurities, which are endogenously produced by the host organism or the host cell line during biotherapeutic manufacturing and production. For recombinant manufacturing of biotherapeutics in a cell, laborious and cost-intensive downstream purification processes are required in order to remove the majority (> 99%) HCPs from the final product. This is not only required for fulfilling the regulatory requirements but the purity of the therapeutic bioproduct is also of utmost importance for a safe use thereof as a drug component. Specifically, HCPs can be highly immunogenic, even when present at very low levels. When HCPs are co-administered with the biotherapeutic agent to a patient, the HCPs in humans can trigger an immune response causing a cytokine storm. Such cytokine storm leads to severe health concerns and, if untreated, can be fatal for the patient.

Typically, HCPs have to be removed during downstream processing (DSP). Exactly the purification steps necessary to obtain the relevant levels of purity in the ultimate biotherapeutic product by removing HCPs, however, significantly contribute to the overall production costs. Purification inherently demands cost-intensive steps conducted under controlled manufacturing conditions that also lead to a decreased product yield.

Moreover, even though current DSP is already rather efficient, a residual amount of HCPs, which are difficult-to-remove, can still remain in the final and commercially distributed biopharmaceutical product. Exemplary biopharmaceuticals, in which such residual HCPs pose a problem, include antibodies, e.g., monoclonal antibodies (mAbs), other therapeutic proteins or peptides, vaccines of any kind, and antibody-drug-conjugates (ADCs). Such residual HCPs can also trigger the immune system and cause severe adverse effects. Moreover, HCPs in biopharmaceuticals can affect their efficacy. For example, residual HCPs have been shown to either degrade the biopharmaceutical itself or excipients in the administered composition, which leads to a reduced shelf life. Furthermore, such degradation can inactivate the biopharmaceutical or generate immunogenic side-products. Consequently, regulatory agencies such as the FDA consider the HCP-content as a critical quality attribute, requiring removal to an acceptable residual material levels as applicable under the pertinent contamination controls following good manufacturing practice (GMP) standards, as measured by highly sensitive analytical methods.

Recent approaches usually aim at reducing HCP at the cell culture production level, which is referred to as upstream processing (USP), but often focus on one target HCP the expression and thus the final HCP-load of which is to be reduced. Specifically, US 10,570,397 B2 and US 9,932,591 B2 aim at reducing one widely known HCP named lipoprotein lipase (LPL) at the USP level by interfering RNA or knocking out at least one copy of the gene encoding endogenous LPL in the host cell. This results in reduced levels if LPL during upstream production. US 2016/251411 A1 discloses a recombinant host cell, wherein the coding sequence of a polynucleotide encoding a phospholipase B-like 2 (PLBD2) protein is modified to thereby reduce the amount of PLBD2, known as abundant HCP, as undesired by-product during recombinant biotherapeutics production.

WO 2022/225880 A1 discloses long lists of potential proteins endogenous to CHO cells the expression of which may be reduced or eliminated to particularly to reduce the expression of endogenous retrovirus-like particles by mammalian cells representing a critical contaminant to be removed during downstream processing.

So far, however, systematic approaches are missing that specifically define and evaluate the most critical HCP pattern in a host cell in a first step to establish, test and provide a generally applicable platform host cell, preferably a modified CHO cell, that has a significantly reduced HCP profile as undesirable co-purifying contaminants whilst simultaneously maintaining or even improving the overall product yield and upstream and downstream processing parameters during recombinant biotherapeutic production in a mammalian cell, preferably a CHO cell.

Consequently, there is still a great need to reduce the content of HCPs in the biopharmaceutical product. It is thus an object of the present invention to reduce the content of HCPs or even eliminate one or more HCPs in the biopharmaceutical product. It is a further object to reduce the content of HCPs or even eliminate one or more HCPs in the biopharmaceutical product at the USP level.

### Summary of the Invention

The present invention tackles the above problems and objects by providing genetically optimized CHO cell lines which are characterized by specific knock-out patterns of targeted HCP or HCP panels resulting in improved impurity profiles at the same time guaranteeing host cell stability and integrity as measured by upstream processing performance. Particularly, the present invention provides a generally applicable host cell, and multiplexing methods of generating the same, wherein the host cell is characterized by a genetic background guaranteeing a much lower HCP profile suitable for producing biotherapeutics at desirable yields in a cost-efficient way.

In a first aspect, there is provided a CHO cell with a modified host cell protein (HCP) profile characterized by a reduced load of at least one endogenous protein being a difficult-to-remove HCP (drHCP) and/or an abundant core HCP (acHCP), wherein said CHO cell comprises: at least one modification in at least one endogenous coding sequence coding for at least one drHCP and/or acHCP selected from the group summarized in **Table 1,** or any homologue, orthologue, or paralogue thereof.

In a second aspect, there is provided a CHO cell with a modified host cell protein (HCP) profile characterized by a reduced load of at least one endogenous protein being a difficult-to-remove HCP (drHCP) and/or an abundant core HCP (acHCP), wherein said CHO cell comprises: two or more modifications in two or more coding sequences coding for two or more drHCPs and/or acHCPs defined / shown in Table 2, 3, 4 or 5, or any homologue, orthologue, or paralogue thereof.

Further, there is provided a CHO cell according to the first or second aspect, wherein at least one, or the two or more modification(s) affect(s) a drHCP, preferably wherein the at least one, or the two or more modification(s) affect(s) one, two, or more protein(s) being both classified as a drHCP and as an acHCP, or wherein said at least one endogenous protein has an amino acid sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, respectively, preferably wherein said at least one endogenous protein has an amino acid sequence corresponding to SEQ ID NO: 2, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 2.

Further, in one embodiment of all aspects disclosed herein, there is provided a CHO cell according to any of the previous aspects or embodiments, wherein said at least one modification leads to the reduced transcription and/or reduced functional expression of said endogenous protein(s) thereby lowering the total content of drHCPs and/or acHCPs.

In another embodiment, there is provided a CHO cell according to any of the previous aspects or embodiments, wherein said CHO comprises at least one recombinant gene encoding at least one recombinant protein of interest and/or encoding at least one recombinant RNA molecule of interest, preferably wherein said at least one recombinant protein of interest is a therapeutic molecule.

In another embodiment, there is provided a CHO cell according to any of the previous aspects or embodiments, wherein said at least one modification is selected from the group consisting of at least one insertion, at least one deletions, and at least one substitution, including a base edit, or any combination thereof, preferably wherein said at least one modification is present in exon 1 of the respective endogenous coding sequence, and/or wherein said at least one modification in said coding sequence is a frame-shift mutation or a point mutation, the point mutation resulting in a stop codon.

In yet another embodiment, there is provided a CHO cell according to any of the previous aspects or embodiments, wherein said at least one modification in said endogenous coding sequence(s) allows for an optimized downstream processing and/or guarantees a reduced load of total HCPs, (i) wherein the CHO cell comprises at least one recombinant non-endogenous gene encoding at least one recombinant protein or RNA of interest and wherein said optimized downstream processing is characterized by a reduced load of total HCPs in the recombinant protein or RNA of product of interest, wherein the total load of HCPs is reduced by at least 0.1%, at least 0.2%, at least 0.3%, at least 0.4%, at least 0.5%, at least 0.6%, at least 0.7%, at least 0.8%, at least 0.9%, preferably at least 1%, at least 1.25%, at least 1.5%, at least 1.75%, at least 2%, at least 2.25%, at least 2.5%, at least 2.75%, at least 3%, at least 3.25%, at least 3.5%, at least 3.75%, at least 4%, at least 4.5%, more preferably at least 5%, at least 5.5, at least 6%, at least 6.5%, at least 7%, at least 7.5%, at least 8%, and even more preferably at least 9%, at least 10%, at least 15%, at least 20%, or at least 25% as determined by measuring the relative concentration of total HCPs in ng/mL per sample obtained in a modified CHO cell in comparison to a reference sample obtained from a CHO wild-type cell, and/or (ii) wherein said at least one modification in said endogenous coding sequence(s) leads to an improved upstream processing performance, wherein said improved upstream processing performance is characterized by an increased concentration of viable cells and/or an increased specific productivity and/or an increased final titer and/or an increased process duration in comparison to a wild-type cell not carrying the at least one modification in its genome. In a further embodiment, there is provided a CHO cell according to any of the preceding embodiments, wherein all alleles of said at least one endogenous coding sequence comprise at least one or more of said modification(s).

Further there is provided a CHO cell according to any of the previous aspects or embodiments, wherein said at least one endogenous protein has an amino acid sequence corresponding to any of the amino acid sequences according to SEQ ID NOs: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, and 128, or corresponding to an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to any one of the sequences according to SEQ ID NOs: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126127, and 128.

In another embodiment, there is provided a CHO cell according to any of the previous aspects or embodiments, wherein the CHO cell comprises at least one modification in at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty or more different HCP encoding sequences, wherein each HCP is preferably selected from an acHCP and/or a drHCP.

In a further aspect, there is provided a method for the production of at least one modified CHO cell according to previous aspects or embodiments comprising the steps: (i) providing at least one CHO cell comprising at least one endogenous target nucleic acid segment; (ii) providing at least one genome or transcriptome editing agent comprising at least one RNAi agent, or at least one site-directed endonuclease, preferably being selected from a meganuclease, a ZFN, a TALEN, a CRISPR-nuclease, or a nickase or nuclease-dead variant therefrom, or a nucleic acid molecule encoding the same, and optionally in case of a CRISPR-nuclease: providing at least one suitable, functional guide RNA molecule, or a nucleic acid molecule encoding the same; (iii) introducing into said at least one CHO cell the at least one genome editing agent of step (ii); (iv) obtaining at least one modified CHO cell comprising at least one modification in said at least one endogenous target nucleic acid segment according to step (i); (v) optionally: selecting a further target nucleic acid segment and repeating steps (i) to (iv) at least one time to obtain at least one modified CHO cell comprising at least one further modification in a further endogenous target nucleic acid segment.

In yet another aspect, there is provided a method for the production of at least one recombinant molecule, preferably at least one recombinant protein, of interest comprising the steps: (a) providing at least one modified CHO cell of as defined in the previous aspects or embodiments, wherein the at least one modified CHO cell comprises at least one recombinant gene encoding at least one recombinant protein, DNA or RNA of interest; (b) culturing said at least one target cell in a culture medium such that at least one recombinant molecule of interest is transcribed and/or translated; (c) harvesting said at least one recombinant molecule of interest; (d) purifying and/ or decontaminating said at least one recombinant molecule, preferably the at least one recombinant protein of interest.

In one additional aspect, there is provided the use of at least one modified CHO cell according to any of the previous aspects or embodiments for the production of at least one pharmaceutical drug.

In yet a further aspect, there is provided a kit comprising (a) at least one modified CHO cell according to any of the previous aspects or embodiments, the CHO cell or the kit comprising: (b.i) at least one nucleic acid molecule suitable for expressing at least one recombinant molecule of interest, preferably at least one recombinant protein of interest; optionally means for introducing the same into the CHO cell genome, or alternatively (b.ii) at least one nucleic acid molecule encoding at least one recombinant molecule of interest, preferably at least one recombinant protein of interest; optionally means for introducing the same into the CHO cell genome; or alternatively (b.iii) at least one nucleic acid molecule suitable for transcribing at least one recombinant RNA molecule of interest; optionally means for introducing the same into the CHO cell genome; or alternatively (b.iv) at least one nucleic acid molecule encoding at least one recombinant RNA molecule of interest; optionally means for introducing the same into the CHO cell genome; and optionally (c) culture medium suitable for the at least one modified CHO cell according to step (a) (i) to replicate the at least one nucleic acid molecule according to any of the steps (b.i), (b.ii), (b.iii), and (b.iv) endogenously or exogenously; and (ii.a) to express the at least one recombinant protein of interest according to step (b.i) and/or (b.ii), and/or to transcribe the at least one recombinant RNA molecule of interest according to step (b.iii) and/or (b.iv).

### Brief Description of Figures

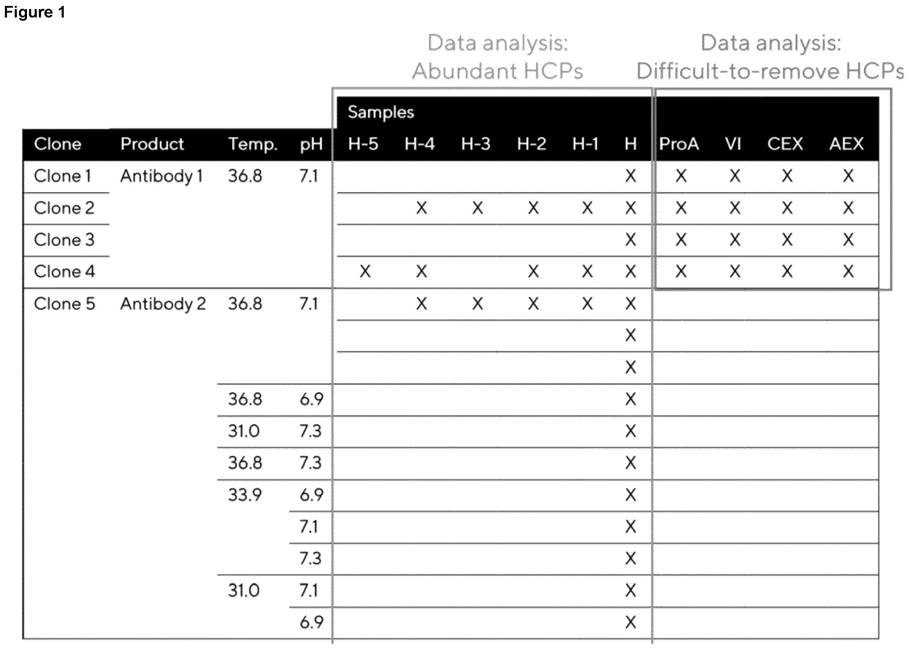

**Figure 1** shows an overview of samples measured by SWATH LC-MS and HCP profiles analyzed to define relevant abundant and difficult-to-remove HCPs as further described in **Example 1.** "H" means harvest. "H-5" thus means 5 days before harvest etc. pp. To obtain a comprehensive and comparable data set, several clones were tested producing different antibody constructs. ProA = Protein A column. VI = virus inactivation step. CEX = cation exchange chromatography. AEX = anion exchange chromatography.

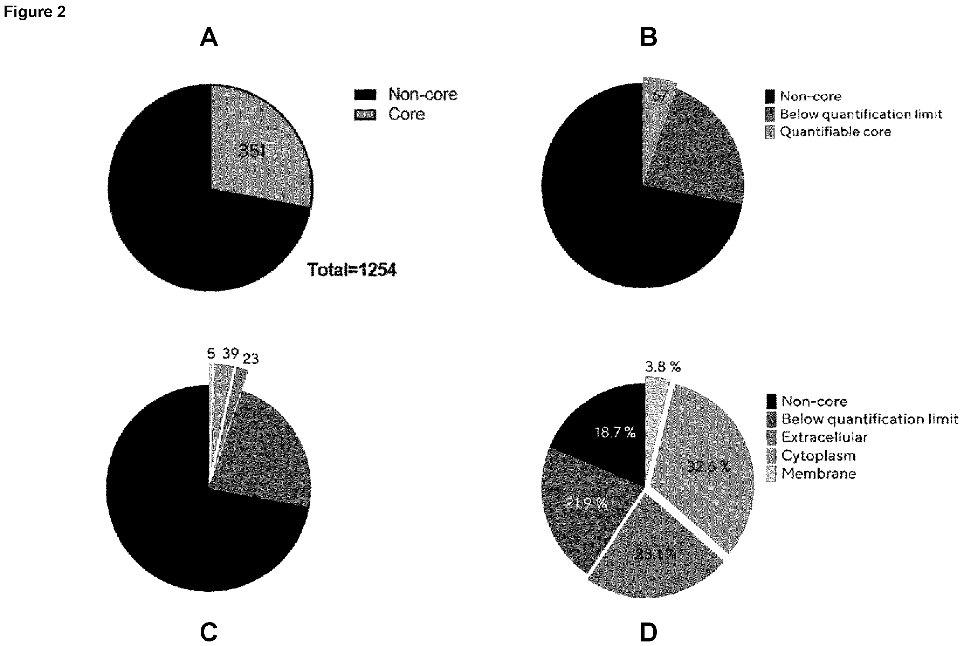

**Figure 2A** to **D (****Fig. 2A** to **D)** gives a schematic overview on the identification process, classification and quantities of core HCPs. **Fig. 2A****:** first round of analysis classifying the 1,254 HCPs tested into core and non-core. **Fig. 2B****:** definition of the cluster of core HCPs quantifiable under the settings chosen characterized further (67). **Fig. 2C****:** further characterization of the 67 quantifiable HCPs of **Fig. 2B****.** Black = Non-core; grey = below LLOQ, 5 = 5 HCPs with membrane localization; 23 = 23 HCPs extracellular; 5 = 5 HCPs with membrane localization (in sum: 67 quantifiable core of **Fig. 2B**). **Fig. 2D****:** shows the percentage of the 67 quantifiable core HCPs of **Fig. 2** **B** and **C** in relation to all 1,254 HCPs analyzed. See also Example 2 for further explanations.

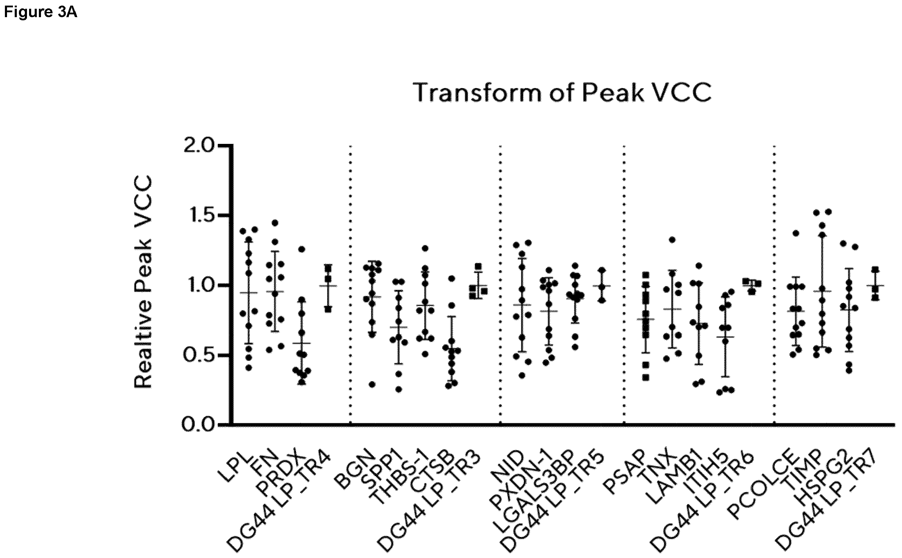

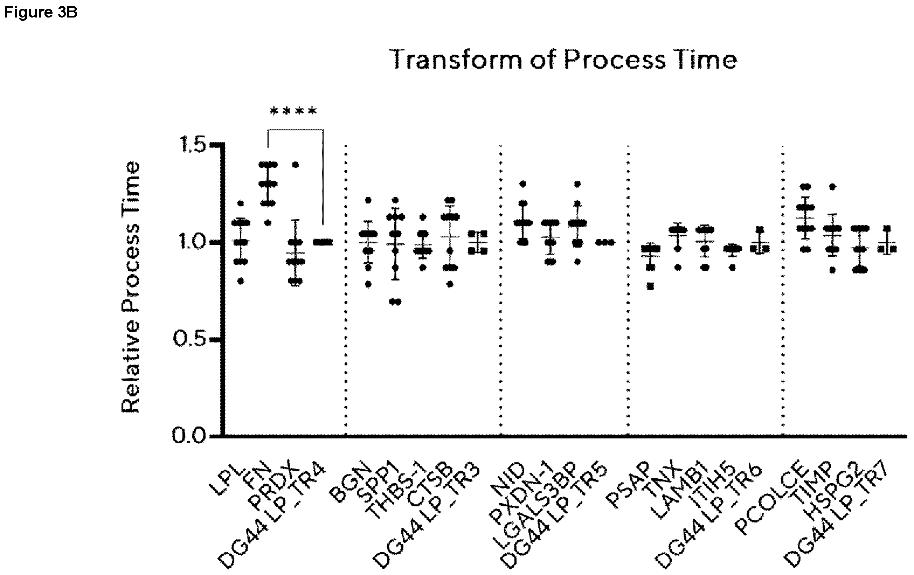

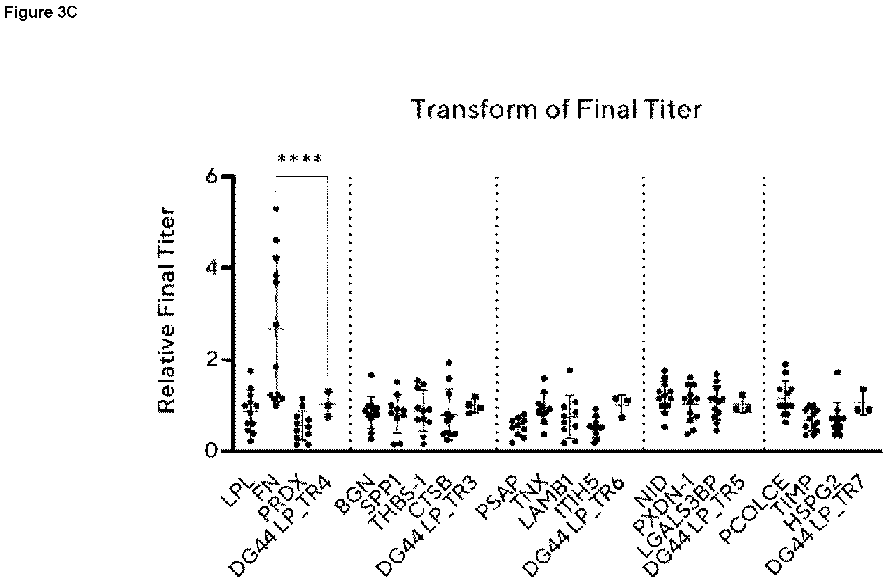

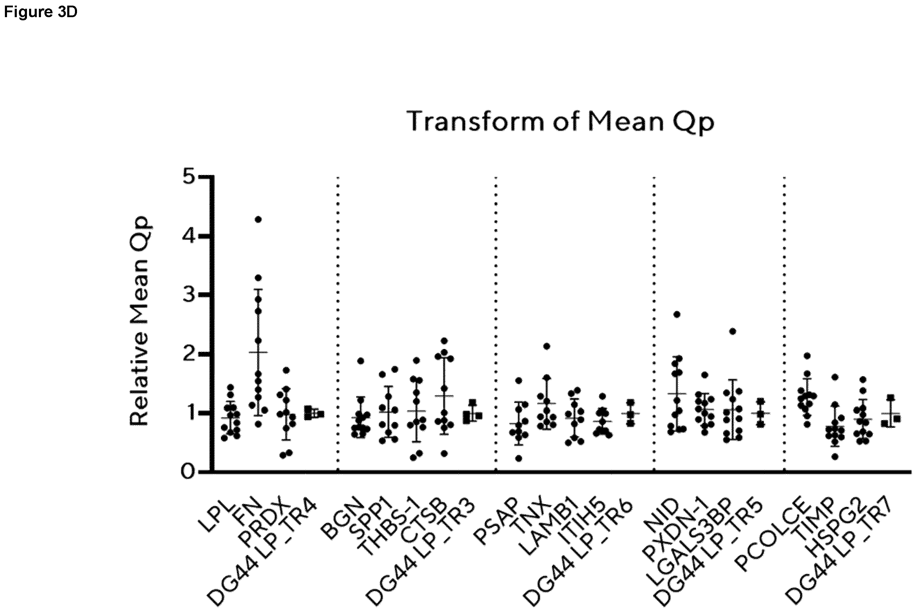

**Figure 3A** to **D (****Fig. 3A** to **D)** shows: **Fig. 3A** the transform of Peak VCC (= viable cell concentration) by plotting the relative peak VCC (y-axis) determined for the generated clones detailed in the Examples (cf. **Example 3)** for the HCP targets as well as a respective CHO DG44 wild-type (WT) controls (TR4, TR3, TR5, TR6 and TR7, respectively - see also **Example 3** for further explanations). Fed-Batch endpoint is shown. **Fig. 3B****:** Transform of process time. **Fig. 3C****:** Transform of Final Titer on harvest day. **Fig. 3D****:** Transform of mean Qp. A process ends once the viability falls below a pre-set threshold. Here, final viability, final titer [g/L] and final Qp [pg/c/d] are relevant. The cell specific productivity Qp is calculated using the cumulative integral of viable cell concentration (IVCC [×10⁵ cells*d/ml]) and titer for each day, respectively. Therefore, IVCC, process time and Qp describe the course of the bioprocess and its productivity. From the Qp, mean Qp is calculated, averaged over the time of cultivation. (Brown-Forysthe and Welch ANOVA test performed, Dunnett's test, alpha = 0.05)

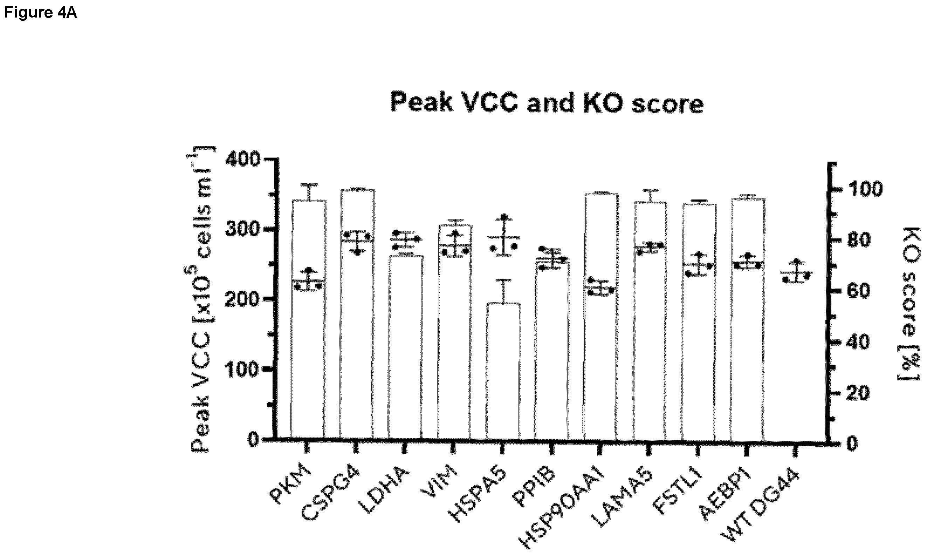

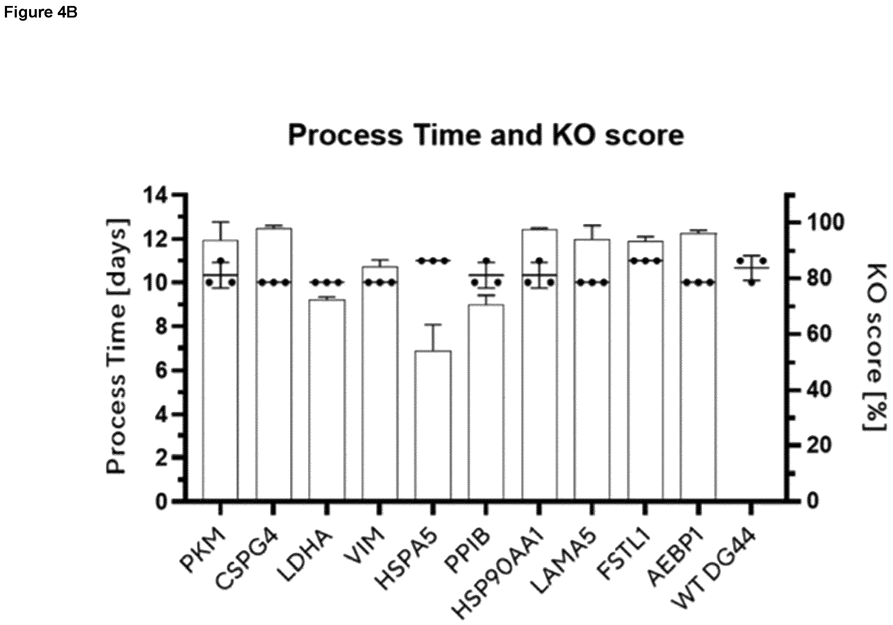

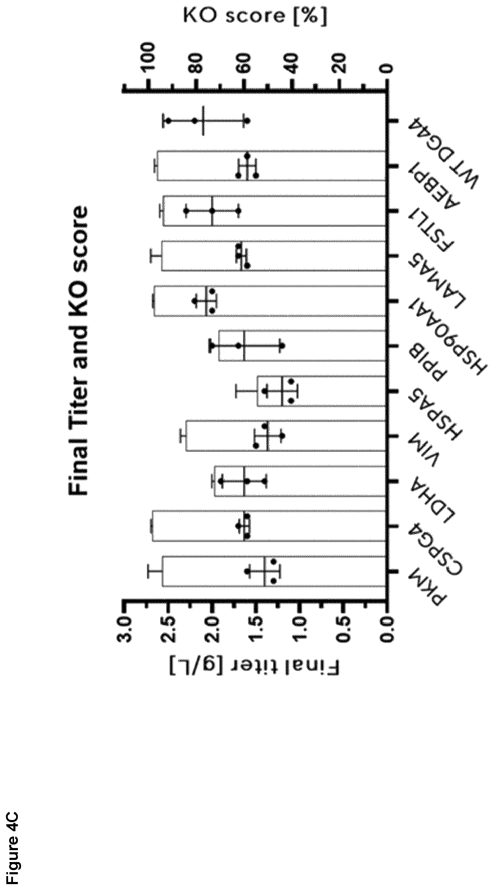

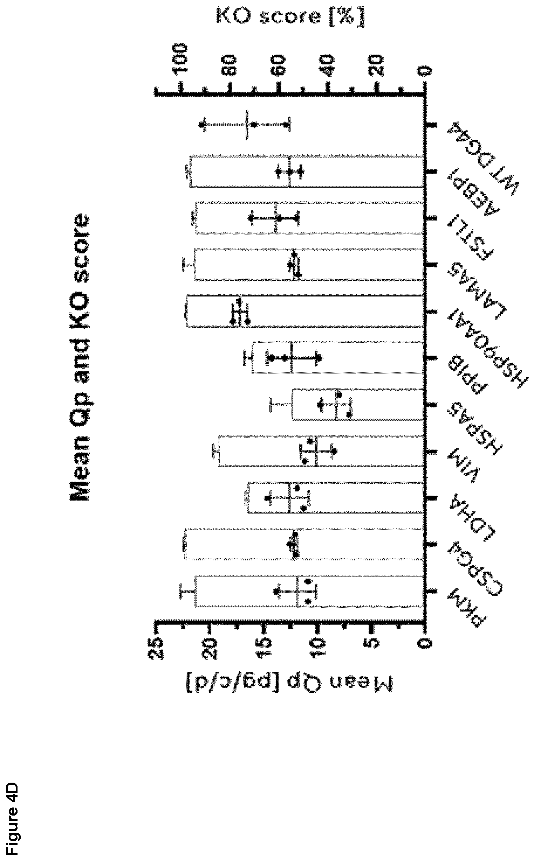

**Figure 4 (Fig. 4A** to **D)** shows the results of the experiments detailed in **Example 4** below, always measured for the 10 target HCPs knocked-out as detailed in this Example and the wild-type (WT) control. **Fig. 4A****:** shows the peak VCC (viable cell concentration in [×10⁵ cells*/ml]) and the KO score (in %). **Fig. 4B****:** shows the process time (in days) and the KO score (in %). **Fig. 4C****:** shows the final titer (in g/L) and the KO score (in %). **Fig. 4D****:** shows final Qp [pg/c/d] and the KO score (in %). The KO score is shown in bars, the further parameter according to **Fig. 4A to D** is reflected by the dots and the corresponding standard deviation.

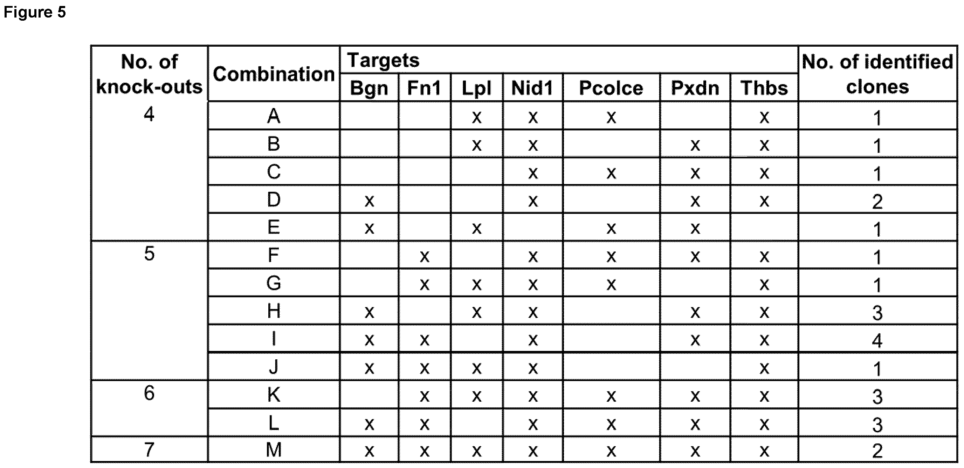

**Figure 5** (cf. **Example 5**) shows a matrix of multiple knock-out combinations (number / no. = 4, 5, 6, or 7) of the seven targets tested in **Example 5** and the number of identified clones.

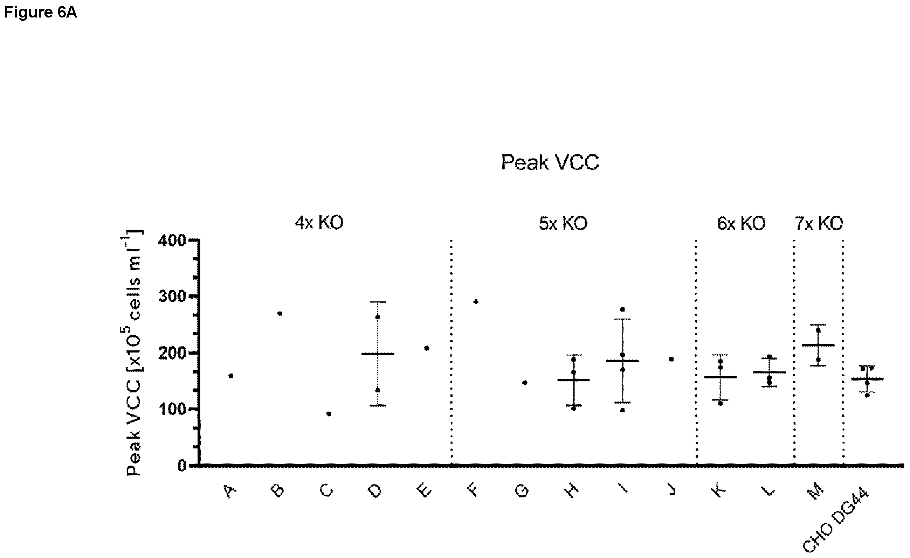

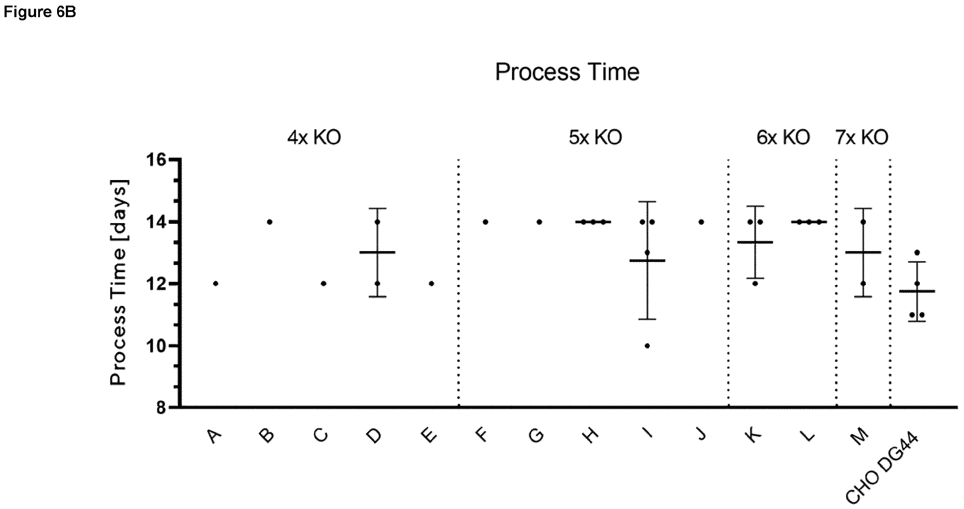

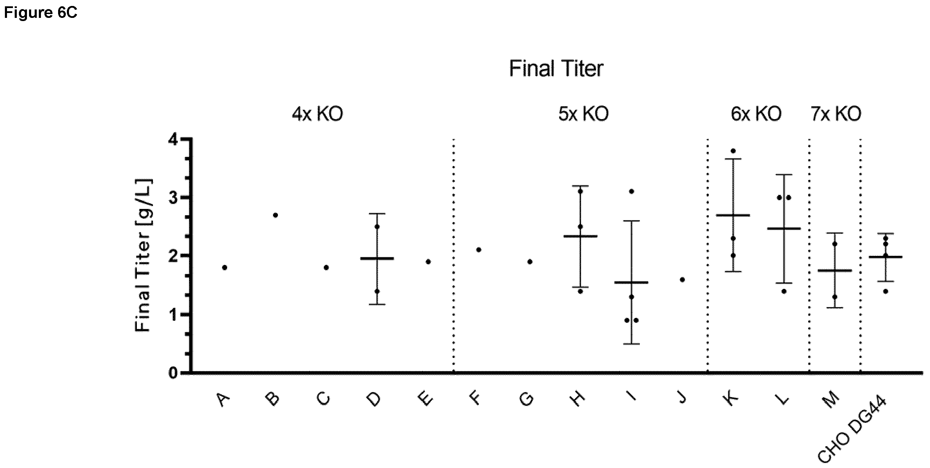

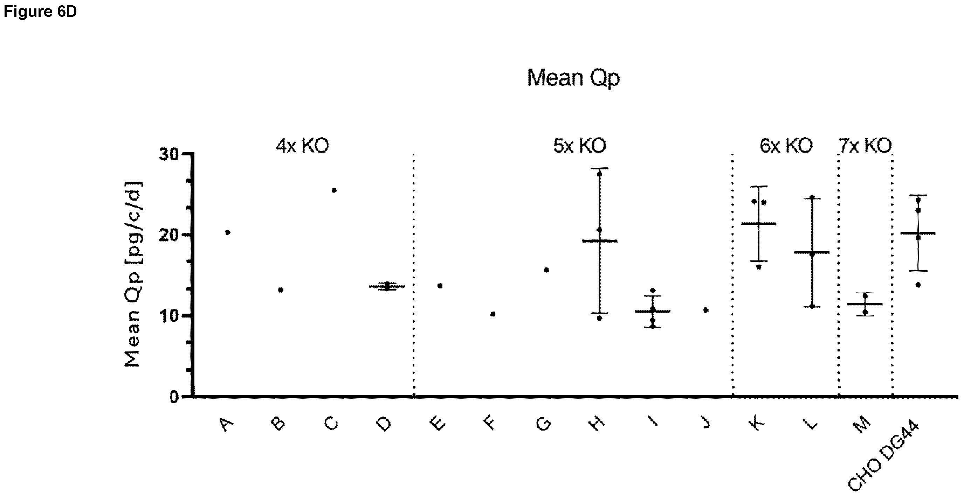

**Figure 6 (Fig. 6A** to **D)** shows the results of the experiments detailed in **Example 5.** All data are presented for the 4-fold, 5-fold, 6-fold or 7-fold KO as detailed in the overview matrix in **Figure 5****.** CHO DG44 served as control for all assays. **Fig. 6A****:** shows the peak VCC (viable cell concentration in [×10⁵ cells*/ml]). **Fig. 6B****:** shows the process time (in days). **Fig. 6C****:** shows the final titer (in g/L). **Fig. 46:** shows the mean Qp [pg/c/d].

### Brief Description of Sequences

| **SEQ ID NO:** | **Description** |
|---|---|
| 1 | G3HHV4_Thrombospondin-1 (=THBS-1) |
| 2 | G3I1V3_Fibronectin (= FN) |
| 3 | G3HPV7_Histone H4 |
| 4 | G3H3Q1_Pyruvate kinase (=PKM) |
| 5 | G3HNJ3_Clusterin |
| 6 | G3GYP9_Peroxiredoxin-1 (= PRDX-1) |
| 7 | P17244_Glyceraldehyde-3-phosphate dehydrogenase |
| 8 | G3GVD0_Actin beta |
| 9 | G3I1Y9_Sulfated glycoprotein 1 (=PSAP) |
| 10 | G3H0E4_Chondroitin sulfate proteoglycan 4 (=CSPG4) |
| 11 | G3I255_L-lactate dehydrogenase (=LDHA) |
| 12 | G3HIM1_Basement membrane-specific heparin sulfate proteoglycan core protein (=HSPG-2) |
| 13 | G3IAQ0_phosphopyruvate hydratase |
| 14 | A0A3L7IKX6_Lipoprotein lipase (= LPL) |
| 15 | G3HHR3_Vimentin (=VIM) |
| 16 | G3I8R9_Endoplasmic reticulum chaperone (=HSPA5) |
| 17 | P19378_Heat shock cognate 71 kDa protein |
| 18 | G3H533_Peptidyl-prolyl cis-trans isomerase (=PPIB) |
| 19 | P09445_Elongation factor 2 |
| 20 | G3H0L9_Cathepsin B (=CTSB) |
| 21 | P46633_Heat shock protein HSP 90-alpha (=HSP90AA1) |
| 22 | G3I3Y6_Glutathione S-transferase |
| 23 | P62629_Elongation factor 1-alpha 1 |
| 24 | G3I3U5_Nidoqen-1 (=NID or NID-1) |
| 25 | G3GUU5_Transketolase |
| 26 | G3I129_Ubiquitin |
| 27 | G3IBF4_Serine_protease |
| 28 | G3HWE4_Nidogen-1 (=NID or NID-1) |
| 29 | G3HBI1_Peroxidasin (=PXDN-1) |
| 30 | G3HKZ1_14-3-3 protein zeta/delta |
| 31 | G3H8V5_Carboxypeptidase |
| 32 | G3HIQ1_Peptidyl-prolyl cis-trans isomerase |
| 33 | G3IKC3_qlutathione transferase |
| 34 | G3HQM6_Endoplasmin |
| 35 | P50310_Phosphoglycerate kinase 1 |
| 36 | A0A061_HUR7_Biqlycan (=BGN) |
| 37 | G3I1H5_Legumain |
| 38 | G3H3E4_Galectin-3-binding protein (=LGAL3BP) |
| 39 | G3I6T1_Phospholipase B-like |
| 40 | G3INX0_Histone H2B |
| 41 | G3H1W4_Tubulointerstitial nephritis antigen like 1 |
| 42 | G3I278_Laminin subunit beta-1 (=LAMB-1; LAMB1) |
| 43 | G3HBD4_Nucleoside diphosphate kinase |
| 44 | G3GR64_Inter-alpha-trypsin inhibitor heavy chain H5 (=ITIH5) |
| 45 | G3H7I6_Sulfhydryl oxidase |
| 46 | G3IF52_Nucleobindin-2 |
| 47 | Q9EPP7_Cathepsin X |
| 48 | G3HZD1_Tenascin-X (=TNX) |
| 49 | G3IG05_Annexin |
| 50 | G3IBK2_Filamin-A |
| 51 | G3HDT6_Histone H2A type 1 |
| 52 | G3I4H6_Fructose-bisphosphate aldolase |
| 53 | G3HH30_Aldo-keto reductase family 1 member B |
| 54 | G3HCX8_Calreticulin |
| 55 | G3I4Z7_Galectin |
| 56 | G3I4E8_Fatty_acid-binding protein |
| 57 | P68361_Tubulin alpha-1B chain |
| 58 | G3IF80_Nucleolin |
| 59 | G3H8F4_Dystroglycan 1 |
| 60 | G3HLV6_ATP_citrate synthase |
| 61 | G3HMV7_Alpha-L-fucosidase |
| 62 | G3HGW6_Laminin subunit alpha-5 (=LAMA5) |
| 63 | G3HC84_Heat shock protein HSP 90-beta |
| 64 | G3GXD7_Fatty acid synthase |
| 65 | G3HQP8_Tubulin beta chain |
| 66 | G3IKQ9_Osteopontin (=SPP1) |
| 67 | G3IIE7_Procollaqen-lysine, 2-oxoglutarate 5-dioxyqenase |
| 68 | G3IBH0_Metalloproteinase inhibitor 1; Tissue inhibitor of metalloproteinases 1(=TIMP1) |
| 69 | G3IHY5_6-phosphoqluconate dehydrogenase |
| 70 | G3ILK7_Calsyntenin-1 |
| 71 | G3HC31_Protein S100 |
| 72 | G3HKG9_60S acidic ribosomal protein |
| 73 | G3HDQ2_Malate dehydroqenase |
| 74 | G3HLK3_Granulins |
| 75 | G3I664_Procollagen C-endopeptidase enhancer 1 (=PCOLCE) |
| 76 | G3HMA1_Adipocyte enhancer-bindinq protein 1 (=AEBP1) |
| 77 | G3H4T5_Glypican-1 |
| 78 | G3HAI3_Follistatin-related protein 1 (=FSTL1) |
| 79 | G3H0U6_protein disulfide-isomerase |
| 80 | G3IHE5_GTP-binding nuclear protein |
| 81 | G3IEY9_14-3-3 protein theta |
| 82 | G3I973_Hypoxia up-requlated 1 |
| 83 | G3IE48_Elonqation factor 1-gamma |
| 84 | G3HDS3_Histone H2A |
| 85 | G3HPV6_Histone H4 |
| 86 | G3HQX0_40S ribosomal protein |
| 87 | G3I6I6_Tubulin alpha chain |
| 88 | G3IED5_Peptidyl-prolyl cis-trans isomerase A |
| 89 | G3IEG6_Tubulin beta chain |
| 90 | A0A3L7HQ14_Eukaryotic translation initiation factor 3 subunit B |
| 91 | G3HC64_FAD synthase |
| 92 | A0A3L7I8J7_Leucine zipper protein 1 |
| 93 | G3IDD4_Serpin H1 |
| 94 | A0A3L7I8K8_Protein Wnt |
| 95 | G3I8B5_Matrix metalloproteinase-28 |
| 96 | A0A8C2LJ19_Sparc/osteonectin |
| 97 | A0A8C2N4D5_protein disulfide-isomerase |
| 98 | A0A8C2MC57_Transforming growth factor beta |
| 99 | A0A8C2N0F8_Trinucleotide repeat containing 6C |
| 100 | G3GXY1_Beta-catenin-like protein 1 |
| 101 | G3GXZ0_Protein-glutamine gamma-glutamyltransferase 2 |
| 102 | A0A8C2LRW3_Catenin delta 1 |
| 103 | G3HFS5_Transforming growth factor beta |
| 104 | A0A8C2LYC0_Laminin subunit qamma 1 |
| 105 | A0A8C2QIZ6_Dynein cytoplasmic 1 heavy chain 1 |
| 106 | A0A8C2QLM9_Collagen type VII alpha 1 chain |
| 107 | G3H996_Catenin beta-1 |
| 108 | A0A8C2MMI8_A disinteqrin-like and metallopeptidase |
| 109 | A0A8C2M328_ADAM metallopeptidase |
| 110 | A0A8C2M6G9_Trinucleotide repeat containinq 6a |
| 111 | O55058_EGF-containing fibulin-like extracellular matrix protein 2 |
| 112 | A0A8C2LVZ9_Milk fat globule-EGF factor 8 protein |
| 113 | A0A8C2MY83_Latent transforming growth factor beta binding protein 3 |
| 114 | G3ICW1_Osteoclast-stimulating factor 1 |
| 115 | G3HAF4_Splicing factor 3B subunit 3 |
| 116 | A0A8C2M0V0_Transforming growth factor beta 1 induced transcript 1 |
| 117 | G3IHE0_Cyclic AMP-dependent transcription factor |
| 118 | G3IK13_Eukaryotic translation initiation factor 3 subunit C |
| 119 | Q8K3H7_Calreticulin |
| 120 | P38982_40S ribosomal protein SA |
| 121 | G3HW00_Trinucleotide repeat-containing gene 6B protein |
| 122 | A0A8C2M0N5_Catenin alpha like 1 |
| 123 | G3HEY9_Catenin_alpha-1 |
| 124 | A0A8C2M1I7_Collagen alpha-2(V) chain |
| 125 | A0A8C2MW41_Diaphanous related formin 1 |
| 126 | G3H4H3_Calreticulin |
| 127 | G3H6V7_Lipoprotein lipase |
| 128 | G3HSX8_Biglycan |
| 129 | LPL_HDR_gRNA1 all guide RNAs: crRNA provided 5'→3' (without PAM sequence) |
| 130 | LPL_HDR_gRNA2 |
| 131 | LPL_HDR_gRNA3 |
| 132 | CTSB-E2-gRNA1 |
| 133 | CTSB-E2-gRNA2 |
| 134 | CTSB-E2-gRNA3 |
| 135 | LPL2_gRNA1 |
| 136 | LPL2_gRNA2 |
| 137 | LPL2_gRNA3 |
| 138 | Bgn_gRNA1 |
| 139 | Bgn_gRNA2 |
| 140 | Bgn_gRNA3 |
| 141 | Fn1_gRNA1 |
| 142 | Fn1_gRNA2 |
| 143 | Fn1_gRNA3 |
| 144 | PRDX1_gRNA1 |
| 145 | PRDX1_gRNA2 |
| 146 | PRDX1_gRNA3 |
| 147 | Psap_gRNA1 |
| 148 | Psap_gRNA2 |
| 149 | Psap_gRNA3 |
| 150 | Spp1_gRNA1 |
| 151 | Spp1_gRNA2 |
| 152 | Spp1_gRNA3 |
| 153 | Thbs_gRNA1 |
| 154 | Thbs_gRNA2 |
| 155 | Thbs_gRNA3 |
| 156 | A0A3L7HVW3 Nidogen-1 isoform (=NID or NID-1) |

### Definitions

The terms "abundant core HCP" (acHCP), "abundant HCP" and "core HCP" are used interchangeably herein. The terms denote those HCPs that (cf. **Fig. 1** and **Table 4)** occur abundant in a host cell of interest in the sense that the HCP is present at the day of harvest and/or is present throughout the cultivation process until harvest, and particularly at the time point of harvest, in a substantial amount, usually a quantifiable amount. Notably, the definition of a core HCP inherently depends on the fact that is it is present above the Lower Limit of Quantification (LLOQ). Still, the skilled person is aware of the fact that the LLOQ may vary depending on the quantification means and the assay conditions used. Further, the LLOQ may be even lower with more sensitive devices and techniques being developed. Therefore, an acHCP as used herein may also refer to an acHCP below the present quantification limit (cf. **Fig. 2B**), if it fulfills the further criterion of being present substantially throughout the complete cultivation process and particularly at the time of harvest. See also **Example 1.**

The term "antibody" as used herein refers to and encompasses various antibody structures known to the skilled person including, but not limited to, monoclonal antibodies, polyclonal antibodies, monospecific antibodies (for example, antibodies consisting of a single heavy chain sequence and a single light chain sequence, including multimers of such pairings), multi specific antibodies (e.g., bi specific antibodies) and antibody fragments with the proviso that the antibody or fragment shows the desired antigen-binding activity as defined by the structure of the at least one complementarity determining region (CDR) of the variable region(s) of the antibody.

An "antibody fragment" or any "(functional) fragment" of an antibody" as used herein refers to a molecule other than a full-length antibody that comprises at least one portion of an intact antibody that binds the antigen to which the full-length antibody binds. Examples of antibody fragments may include, but are not limited to, Fv, Fab, Fab', Fab'-SH, F(ab')2; diabodies; linear antibodies; single-chain antibody molecules (e.g., scFv, and scFab); single domain antibodies (dAbs); and/or multispecific antibodies which assemble from antibody fragments.

In the context of an "antibody" or a "fragment" thereof, the term "chimeric" refers to an antibody in which a portion of the heavy and/or light chain originates from a particular source or species, while the remainder of the heavy and/or light chain is derived from a different source or species. As it is known to one skilled in the art, the "class" of an antibody refers to the type of constant domain or constant region of its cognate heavy chain. There are five major classes of antibodies: IgA, IgD, IgE, IgG and IgM, and several of these can be further divided into subclasses (isotypes), e.g., IgG1, IgG2, IgG3, IgG4, IgAl, and IgA2. In certain embodiments, the antibody is of the IgG 1.

The term "difficult-to-remove HCP" or "drHCP" as used herein refers to an HCP that is present in a quantifiable amount in at least one sample after harvest and after at least one further purification step. Particularly, the HCP is still present in at least one of the samples shown in the right column in **Fig.** 1, i.e., the HCP cannot be removed by at least one of the purification strategies used after harvest. See also **Example 1** for a further explanation and the list provided in **Table 5.**

The term "downstream processing" or "DSP" as used herein, e.g. in the context of cell cultures, refers to the entirety of process steps required for obtaining a recombinantly produced molecule in purified form from a host cell suspension, wherein the cell harvest as the end of the respective bioprocess marks the starting point of the downstream processing as opposed to the upstream process.

"Harvesting" in this context implies the endpoint of the culture of the host cell for obtaining a recombinant protein, including a recombinant peptide, of interest. Harvesting can also be done continuously throughout a cultivation process, e.g. in perfusion culture.

The term "target nucleic acid segment" as used herein refers to a nucleotide sequence, typically a DNA sequence, which can be subjected to modification using a site-directed endonuclease. Typically, a target nucleic acid segment is part of a genome.

Accordingly, the term "upstream processing" or "USP" as used herein, e.g. in the context of cell cultures refers to the entirety of cultivation steps taking place prior to the cell harvest.

The term "upstream processing performance" as used herein refers to an evaluation of the upstream processing of a given cell culture based on one or more parameters, wherein said parameters may be selected from peak viable cell concentration (VCC), integral viable cell concentration (IVCC), cell specific productivity, the final titer, and total bioprocess time. Generally, high values determined for any of the aforementioned parameters indicate a good upstream processing performance. Other parameters may also be assessed for evaluating the upstream processing performance of a given cell culture.

The term "expression" as used herein refers to the process of transcription (e.g., for a functional gRNA) and translation (to achieve a polypeptide) within a host cell. The level or degree of expression of a product gene to the cognate RNA in a host cell can be determined on the basis of either the amount of corresponding mRNA that is present in the cell or the amount of the protein encoded by the product gene that is produced by the cell. For example, mRNA transcribed from a product gene is desirably quantitated by northern hybridization (see, e.g., Sambrook et al., Molecular Cloning: A Laboratory Manual, pp. 7.3-7.57 (Cold Spring Harbor Laboratory Press, 1989). The polypeptide or protein encoded by a product gene can be quantitated either by assaying forthe biological activity of the protein or by using assays that are independent of such activity, such as western blotting using antibodies that are capable of reacting with the protein (see, e.g., Sambrook et al., Molecular Cloning: A Laboratory Manual, pp. 18.1-18.88 (Cold Spring Harbor Laboratory Press, 1989). A "reduced functional expression" as used herein in turn refers to the reduction and/or elimination of the expression of one or more endogenous products relative to the expression level of the endogenous product(s) in an unmodified cell. A reduction of expression may comprise a reduction and/or complete elimination of the functional endogenous product (on RNA and/or protein level).

The term "host cell proteins" (HCPs) as used herein refers to endogenous proteins produced by host cell lines, possibly engineered host cell lines, at varying levels. In case Chinese Hamster Ovary (CHO) cells are used as host cell line, host cell proteins (HCPs) are those proteins, which are produced by CHO cells and which are endogenous to CHO cells.

The term "peptibody" as used herein refers to a part or all of an antibody fused to a peptide. Thus, peptibodies are usually characterized by a combination of the activity of the respective peptide with the longer duration of activity of an antibody.

The terms "polypeptide" and "protein" are used interchangeably herein and refer to a contiguous stretch of amino acid sequences defining the primary structure of the polypeptide. A properly folded protein can have structural (e.g., matrix protein), or functional activity, e.g. enzymatic activity / functional activity, or it can have specific binding or recognition properties (e.g., an antibody), or a combination thereof (binding + nuclease activity, e.g. CRISPR nuclease).

The "recombinant protein of interest" as used herein may be any protein, peptide or fragment thereof that can be produced when exogenously added to a host cell, usually as recombinant (i.e., foreign) DNA or RNA, e.g., on a plasmid, as a viral vector, or as transgene, for example, as introduced via genome editing and the like. Further, a recombinant protein may also be a protein encoded by an endogenous gene that was modified via a genome editing technology, including base editing, prime editing, and InDel induced by genome editing and the like. A recombinant protein of interest can be a therapeutic protein, peptide or fragment thereof, or any other protein, peptide or fragment thereof. The recombinant protein may be an antibody or a variant or functional fragment thereof as defined above, or any other prophylactic or therapeutic protein. Any other protein, e.g., a fluorescent protein like EGFP, is also to be understood as recombinant protein, as long as the protein or fragment thereof has been inserted into, has been mutated, and/or has been artificially created in a host cell of interest.

The terms "RNA interference" or "RNAi" or "RNA silencing" or "gene silencing" as used herein interchangeably refer to a gene down-regulation (or knock-down) mechanism meanwhile demonstrated to exist in all eukaryotes. The mechanism was first recognized in plants where it was called "post-transcriptional gene silencing" or "PTGS". In RNAi, small RNAs function to guide specific effector proteins to a target nucleotide sequence by complementary base pairing resulting in degradation of the target. A "gene silencing construct" or "RNAi agent" usually comprises so called "sense" and "antisense" sequences. Sense and antisense sequences are complementary sequences, which are present in reverse orientation in a nucleic acid sequence. If a nucleic acid construct comprises a sense and a corresponding antisense sequence, the two complementary sequences form an RNA double strand upon transcription, which results in an "RNA hairpin". In an RNA hairpin, sense sequences and corresponding antisense sequences, together form a double strand and are separated by an "intervening intron loop sequence" forming the loop of the hairpin structure. An "RNAi agent" as used herein may also comprise more than one sense and antisense pair and form several loops.

A "therapeutic molecule" or "biotherapeutic molecule or agent" as used herein refers to a recombinant protein of interest as defined above, or any other recombinant nucleic acid molecule that has a prophylactic or curative effect when used to treat a subject in need thereof.

Whenever the present disclosure relates to the percentage of identity of nucleic acid or amino acid sequences to each other these values define those values as obtained by using the EMBOSS Water Pairwise Sequence Alignments (nucleotide) programme (www.ebi.ac.uk/Tools/psa/emboss_water/) nucleic acids or the EMBOSS Water Pairwise Sequence Alignments (protein) programme (www.ebi.ac.uk/Tools/psa/emboss_water/) for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a sequence encoding a protein can be "codon-optimized" if the respective sequence is to be used in another organism in comparison to the original organism a molecule originates from.

### Detailed Description

The present invention thus provides CHO cell lines in which single or multiple HCPs are knocked-out in a targeted way resulting in a more favorable HCP profile without significantly compromising the upstream performance (USP) of cells, including primarily titer, growth, overall viability etc. The HCP composition of the such modified cells is improved in a way that allows for simplified, more economic DSP and/or an improved quality of the final product, because: the HCPs that are abundant in the cell culture harvest are knocked-out and/or HCPs that are difficult to remove during DSP are knocked-out and/or HCPs that can have a negative impact on the product quality are knocked out in a concerted way.

The above objects could thus be solved by the inventors of the present invention by first characterizing a specific HCP contamination profile in comparable samples, by then defining HCP targets and target combinations being particularly problematic as contaminants, and by then defining prioritized knock-down and knock-out strategies to provide modified CHO cells and/or modified CHO cell lines, generally applicable for the production of various biotherapeutics, that show a significantly reduced HCP profile whilst at the same time showing cellular integrity and in turn a favorable upstream and downstream processing performance not hampered by the modification(s). To this end, a strategy was provided that allows for the reliable and targeted elimination of certain abundant and difficult-to-remove HCPs in the final biopharmaceutical without influencing the integrity of the host cell during upstream processing.

In a first aspect, a CHO cell is provided with a modified host cell protein (HCP) profile characterized by a reduced load of at least one endogenous protein being a difficult-to-remove HCP (drHCP) and/or an abundant core HCP (acHCP), wherein said CHO cell comprises: at least one modification in at least one endogenous coding sequence coding for at least one drHCP and/or acHCP selected from the group summarized in Table 1 or any homologue, orthologue, or paralogue thereof. In case two Uniprot IDs are provided in the Tables herein to denote a homologue, orthologue, or paralogue etc., the first one mentioned on the top in the respective Uniprot ID field is the one the respective SEQ ID NO: in the subsequent column refers to.

**Table 1**

| **No.** | **Protein name** | **Uniprot ID** | **SEQ ID NO:** |
|---|---|---|---|
| 1 | Thrombospondin-1 (=THBS-1) | G3HHV4 | 1 |
| 2 | Fibronectin | G3I1V3 | 2 |
| | | A0A3L7IDB0 | |
| 3 | Pyruvate kinase | G3H3Q1 | 4 |
| 4 | Glyceraldehyde-3-phosphate dehydrogenase | P17244 | 7 |
| | | G3HMD1 | |
| 5 | Actin beta | G3GVD0 | 8 |
| 6 | Sulfated glycoprotein 1 | G3I1Y9 | 9 |
| 7 | Chondroitin sulfate proteoglycan 4 | G3H0E4 | 10 |
| 8 | L-lactate dehydrogenase | G3I255 | 11 |
| 9 | Basement membrane-specific heparan sulfate proteoglycan core protein (=HSPG2) | G3HIM1 | 12 |
| | | A0A3L7I8L8 | |
| 10 | Phosphopyruvate hydratase | G3IAQ0 | 13 |
| 11 | Vimentin | G3HHR3 | 15 |
| 12 | Endoplasmic reticulum chaperone BiP | G3I8R9 | 16 |
| 13 | Heat shock cognate 71 kDa protein | P19378 | 17 |
| 14 | Peptidyl-prolyl cis-trans isomerase | G3H533 | 18 |
| 15 | Elongation factor 2 | G3HSL4 | 19 |
| | | P09445 | |
| 16 | Cathepsin B (=CTSB) | G3H0L9 | 20 |
| 17 | Heat shock protein HSP 90-alpha | P46633 | 21 |
| 18 | Glutathione S-transferase | G3I3Y6 | 22 |
| 19 | Elongation factor 1-alpha 1 | P62629 | 23 |
| | | G3HH39 | |
| 20 | Transketolase | G3GUU5 | 25 |
| 21 | Ubiquitin | G3I129 | 26 |
| 22 | Serine protease HTRA1 | G3IBF4 | 27 |
| 23 | Peroxidasin | G3HBI1 | 29 |
| | | A0A3L7HCC3 | |
| 24 | 14-3-3 protein zeta/delta | G3HKZ1 | 30 |
| 25 | Carboxypeptidase | G3H8V5 | 31 |
| 26 | Peptidyl-prolyl cis-trans isomerase | G3HIQ1 | 32 |
| 27 | Glutathione transferase | G3IKC3 | 33 |
| 28 | Endoplasmin | G3HQM6 | 34 |
| 29 | Phosphoglycerate kinase 1 | P50310 | 35 |
| 30 | Histone H2B | G3INX0 | 40 |
| 31 | Laminin subunit beta-1 | G3I278 | 42 |
| | | A0A3L7HMC9 | |
| 32 | Nucleoside diphosphate kinase | G3HBD4 | 43 |
| 33 | Inter-alpha-trypsin inhibitor heavy chain H5 (=ITIH5) | G3GR64 | 44 |
| 34 | Sulfhydryl oxidase | G3H7I6 | 45 |
| 35 | Nucleobindin-2 | G3IF52 | 46 |
| 36 | Cathepsin X | Q9EPP7 | 47 |
| 37 | Tenascin-X | G3HZD1 | 48 |
| 38 | Filamin-A | G3IBK2 | 50 |
| 39 | Histone H2A type 1 | G3HDT6 | 51 |
| 40 | Fructose-bisphosphate aldolase | G3I4H6 | 52 |
| 41 | Aldo-keto reductase family 1 member B | G3HH30 | 53 |
| 42 | Calreticulin | G3HCX8 | 54 |
| 43 | Galectin | G3I4Z7 | 55 |
| | | A0A3L7I2M5 | |
| 44 | Fatty acid-bindinq protein | G3I4E8 | 56 |
| 45 | Tubulin alpha-1B chain | P68361 | 57 |
| 46 | Nucleolin | G3IF80 | 58 |
| 47 | Dystroglycan 1 | G3H8F4 | 59 |
| 48 | ATP citrate synthase | G3HLV6 | 60 |
| 49 | Alpha-L-fucosidase | G3HMV7 | 61 |
| 50 | Laminin subunit alpha-5 (= LAMA5) | G3HGW6 | 62 |
| 51 | Heat shock protein HSP 90-beta | G3HC84 | 63 |
| 52 | Tubulin beta chain | G3HQP8 | 65 |
| | steopontin (=SPP1) | G3IKQ9 | 66 |
| 53 | | | |
| 54 | Procollagen-lysine,2-oxoglutarate 5-dioxygenase 1 | G3IIE7 | 67 |
| 55 | 6-phosphogluconate dehydrogenase, decarboxylating | G3IHY5 | 69 |
| 56 | Calsyntenin-1 | G3ILK7 | 70 |
| 57 | Protein S100 | G3HC31 | 71 |
| 58 | 60S acidic ribosomal protein P0 | G3GU76 | 72 |
| | | G3HKG9 | |
| 59 | Malate dehydrogenase | G3HDQ2 | 73 |
| 60 | Granulins | G3HLK3 | 74 |
| 61 | Procollagen C-endopeptidase enhancer 1 | G3I664 | 75 |
| 62 | Adipocyte enhancer-binding protein 1 | G3HMA1 | 76 |
| 63 | Glypican-1 | G3H4T5 | 77 |
| | | A0A3L7IDU1 | |
| 64 | Follistatin-related protein 1 | G3HAI3 | 78 |
| 65 | Protein disulfide-isomerase | G3H0U6 | 79 |
| 66 | GTP-binding nuclear protein | G3IHE5 | 80 |
| 67 | 14-3-3 protein theta | G3IEY9 | 81 |
| 68 | Hypoxia up-regulated protein 1 | G3I973 | 82 |
| 69 | Elongation factor 1-gamma | G3IE48 | 83 |
| 70 | Histone H2A | G3HDS3 | 84 |
| 71 | Histone H4 | G3HPV6 | 85 |
| 72 | 40S ribosomal protein | G3HQX0 | 86 |
| 73 | Tubulin alpha chain | G3I6I6 | 87 |
| 74 | Peptidyl-prolyl cis-trans isomerase A | G3IED5 | 88 |
| 75 | Tubulin beta chain | G3IEG6 | 89 |
| 76 | Nidogen-1 isoform | A0A3L7HVW3 | 156 |

In Table 1 above, and further in Tables 2 and 3 below, for some proteins more than one Uniprot ID is given, wherein in each case the first Uniprot ID is usually the preferred one, which is to be considered in case a distinction of one or more proteins listed in any of the tables 1, 2, and 3 from any other protein based on their respective Uniprot ID is required. Still, the skilled person will be aware of the fact that orthologues, homologues and paralogues of a given sequence exist. If the present disclosure, for example, mentions an Lipoprotein lipase ("LPL") sequence originating from Chinese Hamster, any known homologue, orthologue, or paralogue thereof (e.g., SEQ ID NOs: 14 and 127) within a given genus and as available in the relevant database (Chinese hamster genome; CH PICRH (GCF_003668045.3) status 27 January 2021, see https://www.chogenome.org/about.php) is meant by the respective abbreviation of an LPL as well. All Uniprot IDs listed for any of the proteins in Tables 1, 2, and 3 correspond to the Uniprot IDs of the respective proteins on the date of filing of this application. Tables 1, 2, and 3 are to be understood such that they also include all homologues, orthologues, or paralogues of all proteins explicitly contained in any of the Tables 1, 2, and 3.

Biotechnological engineering of CHO cells started by employing conventional homologous recombination-based gene targeting strategies (Yamane-Ohnuki et al., 2004, doi: 10.1002/bit.20151). However, such strategies proved rather ineffective as homologous recombination (HR) occurs several orders of magnitude less frequently as compared to non-homologous end-joining (NHEJ) in mammalian cells (Sedivy and Sharp, 1989). NHEJ is particularly applicable for generating gene disruptions, as it is an imperfect repair process often inducing insertions or deletions at the site of the double-strand break (DSB). However, for generating gene disruptions in a site-specific manner, endonucleases suitable for sequence-specific targeting are required, such as transcription activator-like effector nucleases (TALENs), zinc-finger nucleases (ZNFs), CRISPR/Cas effectors, and meganucleases, which were all successfully applied to mammalian cell, i.e. human or CHO cells (Galetto et al., 2009 DOl: 10.1517/14712590903213669; Santiago et al., 2008 doi.org/10.1073/pnas.0800940105; Miller et al., 2010 DOI: 10.1038/nbt.1755; Hillary and Ceasar,. Mol Biotechnol 65, 311-325 (2023). https://doi.org/10.1007/s12033-022-00567-0).

In order to identify the relevant HCP knock-out targets, various clones producing monoclonal antibodies (mAb) were processed under industrial USP (ambr250 fed-batch process) and DSP (standard ProA capture, virus inactivation, cation exchange [CEX] and anion exchange [AEX] polishing steps) conditions. Samples were taken on harvest days - 5, -4, -3, -2, -1 and on the day of harvest. Purification was performed in a standard cascade starting with protein A, followed by virus inaction, CEX and finally AEX polishing.

In a first round, in total, 1,254 proteins were identified and classified. 351 thereof were characterized to be acHCPs. From these 351 candidates, 67 were identified that were always above the quantification limit (cf. **Table 4** below). These were further grouped into different functional clusters based on the localization of the protein: membrane (2), cytoplasm (39), extracellular (16) and unclear or mixed (10) (cf. **Fig. 2C**). This panel was expanded in a stepwise way to fully cover the CHO HCP profile.

By systematically testing the acHCP and drHCP profile, relevant targets and combinations thereof were determined and, for each target, test knock-outs were created to evaluate the suitability of the protein as knock-out target to avoid lethal or USP hampering knock-outs.

The term "recombinant" as used herein, and as generally defined above, usually refers to molecules, e.g. proteins or nucleic acid molecules, which are non-endogenous to the cells, in which they are expressed. For example, genetic material could have been introduced into a cell, which afterwards can express (transcribe and/or translate) the recombinant molecule(s) of interest.

In one embodiment of all aspects and embodiments as disclosed herein, the modified CHO cell may be selected from, or may be derived from, meaning originating from, a CHO cell variant selected from the group consisting of CHO-K1, CHO-DXB11 (synonymously referred to as CHO-DUKX), CHO-DG44, CHO-S, and CHO-Pro minus, or a CHO GS knock out. Alternatively, the modified CHO cell according to the present invention may also be derived from other CHO cell variants.

In a preferred embodiment, the modified CHO cell may be, or may be derived from a CHO-DG44.

In another preferred embodiment, the modified CHO cell may be, or may be derived from a CHO-K1.

In yet a preferred, the modified CHO cell may be, or may be derived from CHO-DXB11 (synonymously referred to as CHO-DUKX).

In yet another preferred embodiment, the CHO cell may be, or may be derived from a CHO GS knock out cell.

In one preferred embodiment, the modified CHO cell may be derived from CHO-S, or it may be derived from CHO-Pro minus.

In one embodiment, the at least one modification may be present in at least one endogenous coding sequence, which codes for an HCP selected from the group consisting of the proteins with any one of SEQ ID NO: 1, 2, 4, 7, 8, 9, 10, 11, 12, 13, 15, 16, 17, 18, 19, 20, 21, 22, 23, 25, 26, 27, 29, 30, 31, 32, 33, 34, 35, 40, 42, 43, 44, 45, 46, 47, 48, 50, 51, 52, 53, 54, 55, 56, 57, 58, 59, 60, 61, 62, 63, 65, 66, 67, 69, 70, 71, 72, 73, 74, 75, 76, 77, 78, 79, 80, 81, 82, 83, 84, 85, 86, 87, 88, and 89 as listed in Table 1 or any homologue, orthologue, or paralogue thereof.

In one embodiment, the at least one modification may be present in at least one endogenous coding sequence, which codes for a HCP selected from the group consisting of SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, respectively, preferably wherein said at least one endogenous protein has an amino acid sequence corresponding to SEQ ID NO: 2, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 2.

In preferred embodiments at least 2, 3, 4, 5, 6, 7, 8, 9, 10, 11, 12, 13, 14, 15, 16, 17, 18, 19, 20, 21, 22, 23, 24, 25, 26, 27, 28, 29, 30 or more modification of an HCP shown in SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, or any homologue, orthologue, or paralogue thereof the above may be combined with each other, or may be combined with any knock-out of an HCP as shown in Table 1, preferably wherein at least one modification, or at least 2, 3, 4, 5, 6, or 7 of the simultaneous mutations are a knock-out of the sequence shown with SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, or any homologue, orthologue, or paralogue thereof.

In a second aspect, there may be provided a CHO cell with a modified host cell protein (HCP) profile characterized by a reduced load of at least one endogenous protein being a difficult-to-remove HCP (drHCP) and/or an abundant core HCP (acHCP), wherein said CHO cell comprises: two or more modifications in two or more coding sequences coding for two or more drHCPs and/or acHCPs defined / shown in Table 2, 3, 4 or 5, or any homologue, orthologue, or paralogue thereof, preferably wherein said at least one endogenous protein is fibronectin or any homologue, orthologue, or paralogue thereof; and/or preferably wherein said fibronectin has an amino acid sequence corresponding to SEQ ID NO: 2, or an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 2.

**Table 2**

| **No.** | **Protein name** | **Uniprot ID** | **SEQ ID NO:** |
|---|---|---|---|
| 1 | Thrombospondin-1 | G3HHV4 | 1 |
| 2 | Fibronectin | G3I1V3 A0A3L7IDB0 | 2 |
| 4 | Pyruvate kinase | G3H3Q1 | 4 |
| 5 | Clusterin | G3HNJ3 | 5 |
| 6 | Peroxiredoxin-1 | G3GYP9 | 6 |
| 7 | Glyceraldehyde-3-phosphate dehydrogenase | P17244 G3HMD1 | 7 |
| 8 | Actin beta | G3GVD0 | 8 |
| 9 | Sulfated glycoprotein 1 | G3I1Y9 | 9 |
| 10 | Chondroitin sulfate proteoglycan 4 | G3H0E4 | 10 |
| 11 | L-lactate dehydrogenase | G3I255 | 11 |
| 12 | Basement membrane-specific heparan sulfate proteoglycan core protein | G3HIM1 A0A3L7I8L8 | 12 |
| 13 | Phosphopyruvate hydratase | G3IAQ0 | 13 |
| 14 | Lipoprotein lipase (=LPL) | G3H6V7 A0A3L7IKX6 | 127, 14 |
| 15 | Vimentin | G3HHR3 | 15 |
| 16 | Endoplasmic reticulum chaperone BiP | G3I8R9 | 16 |
| 17 | Heat shock cognate 71 kDa protein | P19378 | 17 |
| 18 | Peptidyl-prolyl cis-trans isomerase | G3H533 | 18 |
| 19 | Elongation factor 2 | G3HSL4 P09445 | 19 |
| 20 | Cathepsin B | G3H0L9 | 20 |
| 21 | Heat shock protein HSP 90-alpha | P46633 | 21 |
| 22 | Glutathione S-transferase | G3I3Y6 | 22 |
| 23 | Elongation factor 1-alpha 1 | P62629 G3HH39 | 23 |
| 24 | Nidogen-1 | G3I3U5 | 24 |
| 25 | Transketolase | G3GUU5 | 25 |
| 26 | Ubiquitin | G3I129 | 26 |
| 27 | Serine protease HTRA1 | G3IBF4 | 27 |
| 28 | Nidogen-1 (=NID or NID-1) | G3HWE4 A0A3L7HVW3 | 28, 156 |
| 29 | Peroxidasin | G3HBI1 A0A3L7HCC3 | 29 |
| 30 | 14-3-3 protein zeta/delta | G3HKZ1 | 30 |
| 31 | Carboxypeptidase | G3H8V5 | 31 |
| 32 | Peptidyl-prolyl cis-trans isomerase | G3HIQ1 | 32 |
| 33 | Glutathione transferase | G3IKC3 | 33 |
| 34 | Endoplasmin | G3HQM6 | 34 |
| 35 | Phosphoglycerate kinase 1 | P50310 | 35 |
| 36 | Biglycan | G3HSX8 A0A061HUR7 | 36, 128 |
| 37 | Legumain | G3I1H5 | 37 |
| 38 | Galectin-3-binding protein | G3H3E4 | 38 |
| 39 | Phospholipase B-like | G3I6T1 | 39 |
| 40 | Histone H2B | G3INX0 | 40 |
| 41 | Tubulointerstitial nephritis antigen-like | G3H1W4 | 41 |
| 42 | Laminin subunit beta-1 | G3I278 A0A3L7HMC9 | 42 |
| 43 | Nucleoside diphosphate kinase | G3HBD4 | 43 |
| 44 | Inter-alpha-trypsin inhibitor heavy chain H5 | G3GR64 | 44 |
| 45 | Sulfhydryl oxidase | G3H7I6 | 45 |
| 46 | Nucleobindin-2 | G3IF52 | 46 |
| 47 | Cathepsin X | Q9EPP7 | 47 |
| 48 | Tenascin-X | G3HZD1 | 48 |
| 49 | Annexin | G3IG05 | |
| 50 | Filamin-A | G3IBK2 | 50 |
| 51 | Histone H2A type 1 | G3HDT6 | 51 |
| 52 | Fructose-bisphosphate aldolase | G3I4H6 | 52 |
| 53 | Aldo-keto reductase family 1 member B | G3HH30 | 53 |
| 54 | Calreticulin | G3HCX8 | 54 |
| 55 | Galectin | G3I4Z7 A0A3L7I2M5 | 55 |
| 56 | Adipocyte-type fatty acid-binding protein | G3I4E8 | 56 |
| 57 | Tubulin alpha-1B chain | P68361 | 57 |
| 58 | Nucleolin | G3IF80 | 58 |
| 59 | Dystroglycan 1 | G3H8F4 | 59 |
| 60 | ATP citrate synthase | G3HLV6 | 60 |
| 61 | Alpha-L-fucosidase | G3HMV7 | 61 |
| 62 | Laminin subunit alpha-5 | G3HGW6 | 62 |
| 63 | Heat shock protein HSP 90-beta | G3HC84 | 63 |
| 64 | Fatty acid synthase | G3GXD7 | 64 |
| 65 | Tubulin beta chain | G3HQP8 | 65 |
| 66 | Osteopontin | G3IKQ9 | 66 |
| 67 | Procollagen-lysine,2-oxoglutarate 5-dioxygenase 1 | G3IIE7 | 67 |
| 68 | Metalloproteinase inhibitor 1 | G3IBH0 | 68 |
| 69 | 6-phosphogluconate dehydrogenase, decarboxylating | G3IHY5 | 69 |
| 70 | Calsyntenin-1 | G3ILK7 | 70 |
| 71 | Protein S100 | G3HC31 | 71 |
| 72 | 60S acidic ribosomal protein P0 | G3GU76 G3HKG9 | 72 |
| 73 | Malate dehydrogenase | G3HDQ2 | 73 |
| 74 | Granulins | G3HLK3 | 74 |
| 75 | Procollagen C-endopeptidase enhancer 1 | G3I664 | 75 |
| 76 | Adipocyte enhancer-binding protein 1 =(AEBP1) | G3HMA1 | 76 |
| 77 | Glypican-1 | G3H4T5 A0A3L7IDU1 | 77 |
| 78 | Follistatin-related protein 1 | G3HAI3 | 78 |
| 79 | Protein disulfide-isomerase | G3H0U6 | 79 |
| 80 | GTP-binding nuclear protein Ran | G3IHE5 | 80 |
| 81 | 14-3-3 protein theta | G3IEY9 | 81 |
| 82 | Hypoxia up-regulated protein 1 | G3I973 | 82 |
| 83 | Elongation factor 1-qamma | G3IE48 | 83 |
| 84 | Histone H2A | G3HDS3 | 84 |
| 85 | Histone H4 | G3HPV6 | 85 |
| 86 | 40S ribosomal protein SA | G3HQX0 | 86 |
| 87 | Tubulin alpha chain | G3I6I6 | 87 |
| 88 | Peptidyl-prolyl cis-trans isomerase A | G3IED5 | 88 |
| 89 | Tubulin beta chain | G3IEG6 | 89 |

In one embodiment, the two or more modifications may be independently selected from the group consisting of insertions, deletion and substitutions.

In another embodiment of the present invention, at least one, preferably at least two, preferably at least three, preferably at least four, preferably at least five, preferably at least six, preferably at least seven, preferably at least eight, preferably at least nine, preferably at least ten, preferably at least eleven, preferably at least twelve, preferably at least thirteen, preferably at least fourteen, preferably at least fifteen, preferably at least sixteen, preferably at least seventeen, preferably at least eighteen, preferably at least nineteen, preferably all, of the two or more modifications may be (a) deletion(s).

In a preferred embodiment of the present invention, at least one, preferably at least two, preferably at least three, preferably at least four, preferably at least five, preferably at least six, preferably at least seven, preferably at least eight, preferably at least nine, preferably at least ten, preferably at least eleven, preferably at least twelve, preferably at least thirteen, preferably at least fourteen, preferably at least fifteen, preferably at least sixteen, preferably at least seventeen, preferably at least eighteen, preferably at least nineteen, preferably all, of the two or more modifications may be (a) insertion(s).

In another preferred embodiment of the present invention, at least one, preferably at least two, preferably at least three, preferably at least four, preferably at least five, preferably at least six, preferably at least seven, preferably at least eight, preferably at least nine, preferably at least ten, preferably at least eleven, preferably at least twelve, preferably at least thirteen, preferably at least fourteen, preferably at least fifteen, preferably at least sixteen, preferably at least seventeen, preferably at least eighteen, preferably at least nineteen, preferably all, of the two or more modifications may be (a) substitution(s).

In an exemplary embodiment of the present invention, the modified CHO cell may be characterized by a reduced load of Fibronectin (Uniprot ID: G3I1V3; SEQ ID NO: 2) and at least one, preferably at least two, preferably at least three, preferably at least four, preferably at least five, preferably at least six, preferably at least seven, preferably at least eight, preferably at least nine, preferably at least ten, preferably at least eleven, preferably at least twelve, preferably at least thirteen, preferably at least fourteen, preferably at least fifteen, preferably at least sixteen, preferably at least seventeen, preferably at least eighteen, preferably at least nineteen, other endogenous protein(s) independently selected from the group consisting of the proteins according to any one of SEQ ID NOs: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, , 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 127 or 128, or any homologue, orthologue, or paralogue thereof, or selected from the group consisting of the proteins as listed in any one of Tables 1, 2, 3, 4 or 5, or any homologue, orthologue, or paralogue thereof.

In a preferred embodiment of all aspects disclosed herein relating to the CHO cell according to the present invention, the at least one, or the two or more modification(s) may affect a drHCP, preferably wherein the at least one, or the two or more modification(s) affect(s) one, two, or more protein(s) being both classified as a drHCP and as an acHCP.

In a preferred embodiment of all aspects of the present invention, the two or more modification(s) may affect one, two, or more protein(s) being both classified as a drHCP and as an acHCP independently selected from the group consisting of all proteins listed in Table 3.

**Table 3**

| **No.** | **Protein name** | **Uniprot ID** | **SEQ ID NO:** |
|---|---|---|---|
| 1 | Thrombospondin-1 | G3HHV4 | 1 |
| 2 | Fibronectin | G3I1V3 A0A3L7IDB0 | 2 |
| 3 | Pyruvate kinase | G3H3Q1 | 4 |
| 4 | Clusterin | G3HNJ3 | 5 |
| 5 | Peroxiredoxin-1 | G3GYP9 | 6 |
| 6 | Glyceraldehyde-3-phosphate dehydrogenase | P17244 G3HMD1 | 7 |
| 7 | Actin beta | G3GVD0 | 8 |
| 8 | Sulfated glycoprotein 1 | G3I1Y9 | 9 |
| 9 | L-lactate dehydrogenase | G3I255 | 11 |
| 10 | Basement membrane-specific heparan sulfate proteoglycan core protein | G3HIM1 A0A3L7I8L8 | 12 |
| 11 | Phosphopyruvate hydratase | G3IAQ0 | 13 |
| 12 | Lipoprotein lipase | G3H6V7 A0A3L7IKX6 | 127, 14 |
| 13 | Endoplasmic reticulum chaperone BiP | G3I8R9 | 16 |
| 14 | Heat shock cognate 71 kDa protein | P19378 | 17 |
| 15 | Elongation factor 2 | G3HSL4 P09445 | 19 |
| 16 | Cathepsin B | G3H0L9 | 20 |
| 17 | Heat shock protein HSP 90-alpha | P46633 | 21 |
| 18 | Glutathione S-transferase | G3I3Y6 | 22 |
| 19 | Elongation factor 1-alpha 1 | P62629 G3HH39 | 23 |
| 20 | Nidogen-1 | G3I3U5 | 24 |
| 21 | Serine protease | G3IBF4 | 27 |
| 22 | Carboxypeptidase | G3H8V5 | 31 |
| 23 | Endoplasmin | G3HQM6 | 34 |
| 24 | Phosphoglycerate kinase 1 | P50310 | 35 |
| 25 | Tubulointerstitial nephritis antigen-like | G3H1W4 | 41 |
| 26 | Annexin | G3IG05 | 49 |
| 27 | Galectin | G3I4Z7 A0A3L7I2M5 | 55 |

In one embodiment of all aspects as disclosed herein, the at least one modification may be present in at least one endogenous coding sequence, which codes for a HCP selected from the group consisting of Thrombospondin-1 (THBS1, Uniprot ID: G3HHV4; SEQ ID NO: 1), Fibronectin (FN/FN1, Uniprot ID: G3I1V3; SEQ ID NO: 2), Sulfated glycoprotein 1 (PSAP, Uniprot ID: G3I1Y9; SEQ ID NO: 9), and/or Chondroitin sulfate proteoglycan 4 (=CSPG4) (Uniprot ID: G3H0E4; SEQ ID NO: 10). These targets were acHCPs and knocking-out at least one or all four of them favourably reduced the overall HCP burden in a final purified preparation. In one embodiment, THBS1 and/or FN1 and/or PSAP and/or CSPG4 represent a knock-out target, alone, or in dual, triple, or quadruple combination. Further, THBS1 and/or FN1 and/or PSAP and/or CSPG4m, alone or in combination with each other, represent a suitable panel of basic HCP modifications that can be combined with at least one further HCP knock.-out, preferably a knock-out of at least one further acHCP and/or drHCP.

In preferred embodiments, the at least one modification may be present in at least one endogenous coding sequence, which codes for a HCP being highly abundant (acHCPs). **Table 4** below summarizes a list of highly abundant acHCPs, for which the mean relative concentration, named "MRC" in **Table 4** below, was calculated for each individual HCP by determining the mean value of all measured concentrations (in ng/mL) over the sum of all probes measured. All mean values were added to each other and for each HCP, the mean value was divided by the mean HCP total concentration. The resulting value corresponds to the "mean relative concentration" or MRC as used herein.

Modifying, i.e., reducing, the amount of at least one acHCP expressed in a cell with a high mean relative concentration meaning a concentration above 0.05%, preferably above 0.1%, even more preferably 0.15% and above will lead to a significant decrease of the overall HCP load. This is particularly true for acHCPs with a very high mean relative concentration meaning a concentration above 0.5%, preferably above 1%, even more preferably with a mean relative concentration of 1.5% and above.

**Table 4**

| Protein name | Uniprot ID | Location | MRC |
|---|---|---|---|
| Thrombospondin-1 | G3HHV4 | extracellular | 6,54% |
| Fibronectin | G3I1V3 A0A3L7IDB0 | extracellular | 4,53% |
| Histone H4 | G3HPV7 | intracellular | 2,72% |
| Pyruvate kinase | G3H3Q1 | intracellular | 2,68% |
| Clusterin | G3HNJ3 | extracellular | 2,49% |
| Peroxiredoxin-1 | G3GYP9 | intracellular | 2,36% |
| Glyceraldehyde-3-phosphate dehydrogenase | P17244 G3HMD1 | intracellular | 2,14% |
| Actin beta | G3GVD0 | intracellular | 1,85% |
| Sulfated glycoprotein 1 | G3I1Y9 | extracellular | 1,83% |
| Chondroitin sulfate proteoglycan 4 | G3H0E4 | plasma membrane | 1,83% |
| L-lactate dehydrogenase | G3I255 | intracellular | 1,55% |
| Basement membrane-specific heparan sulfate proteoglycan core protein | G3HIM1 A0A3L7I8L8 | plasma membrane | 1,54% |
| Phosphopyruvate hydratase | G3IAQ0 | intracellular | 1,46% |
| Lipoprotein lipase | G3H6V7 A0A3L7IKX6 | extracellular | 1,31% |
| Vimentin | G3HHR3 | intracellular | 1,28% |
| Endoplasmic reticulum chaperone BiP | G3I8R9 | intracellular | 1,25% |
| Heat shock cognate 71 kDa protein | P19378 | intracellular | 1,24% |
| Peptidyl-prolyl cis-trans isomerase | G3H533 | intracellular | 1,22% |
| Elongation factor 2 | G3HSL4 P09445 | intracellular | 1,16% |
| Cathepsin B | G3H0L9 | intracellular | 1,15% |
| Heat shock protein HSP 90-alpha | P46633 | intracellular | 1,11% |
| Glutathione S-transferase | G3I3Y6 | intracellular | 1,00% |
| Elongation factor 1-alpha 1 | P62629 G3HH39 | intracellular | 0,96% |
| Nidogen-1 | G3I3U5 | extracellular | 0,84% |
| Transketolase | G3GUU5 | intracellular | 0,83% |
| Serine protease HTRA1 | G3IBF4 | extracellular | 0,78% |
| Nidogen-1 | G3HWE4 A0A3L7HVW3 | extracellular | 0,76% |
| Peroxidasin | G3HBI1 A0A3L7HCC3 | extracellular | 0,69% |
| 14-3-3 protein zeta/delta | G3HKZ1 | intracellular | 0,69% |
| Carboxypeptidase | G3H8V5 | extracellular | 0,66% |
| Glutathione transferase | G3IKC3 | intracellular | 0,66% |
| Endoplasmin | G3HQM6 | intracellular | 0,58% |
| Phosphoglycerate kinase 1 | P50310 | intracellular | 0,56% |
| Biglycan | G3HSX8 A0A061HUR7 | extracellular | 0,55% |
| Legumain | G3I1H5 | intracellular | 0,52% |
| Galectin-3-binding protein | G3H3E4 | extracellular | 0,50% |
| Phospholipase B-like | G3I6T1 | intracellular | 0,49% |
| Tubulointerstitial nephritis antigen-like | G3H1W4 | intracellular | 0,47% |
| Laminin subunit beta-1 | G3I278 A0A3L7HMC9 | extracellular | 0,44% |
| Nucleoside diphosphate kinase | G3HBD4 | intracellular | 0,43% |
| Inter-alpha-trypsin inhibitor heavy chain H5 | G3GR64 | extracellular | 0,41% |
| Sulfhydryl oxidase | G3H7I6 | extracellular | 0,40% |
| Nucleobindin-2 | G3IF52 | intracellular | 0,39% |
| Cathepsin X | Q9EPP7 | intracellular | 0,39% |
| Tenascin-X | G3HZD1 | extracellular | 0,38% |
| Annexin | G3IG05 | extracellular | 0,38% |
| Filamin-A | G3IBK2 | intracellular | 0,37% |
| Fructose-bisphosphate aldolase | G3I4H6 | intracellular | 0,37% |
| Aldo-keto reductase family 1 member B | G3HH30 | intracellular | 0,37% |
| Galectin | G3I4Z7 A0A3L7I2M5 | extracellular | 0,36% |
| Adipocyte-type fatty acid-binding protein | G3I4E8 | intracellular | 0,35% |
| Tubulin alpha-1B chain | P68361 | intracellular | 0,35% |
| Nucleolin | G3IF80 | intracellular | 0,35% |
| Dystroglycan 1 | G3H8F4 | plasma membrane | 0,33% |
| ATP citrate synthase | G3HLV6 | intracellular | 0,32% |
| Alpha-L-fucosidase | G3HMV7 | intracellular | 0,32% |
| Laminin subunit alpha-5 | G3HGW6 | extracellular | 0,30% |
| Heat shock protein HSP 90-beta | G3HC84 | intracellular | 0,29% |
| Fatty acid synthase | G3GXD7 | intracellular | 0,29% |
| Tubulin beta chain | G3HQP8 | intracellular | 0,28% |
| Procollagen-lysine,2-oxoglutarate 5-dioxygenase 1 | G3IIE7 | intracellular | 0,24% |
| Metalloproteinase inhibitor 1 | G3IBH0 | extracellular | 0,23% |
| Calsyntenin-1 | G3ILK7 | plasma membrane | 0,21% |
| Procollagen C-endopeptidase enhancer 1 | G3I664 | extracellular | 0,17% |
| Adipocyte enhancer-binding protein 1 | G3HMA1 | extracellular | 0,17% |
| Glypican-1 | G3H4T5 A0A3L7IDU1 | plasma membrane | 0,16% |
| Follistatin-related protein 1 | G3HAI3 | extracellular | 0,15% |

Further, in particular embodiments, it may be preferable that the at least one modification is present in at least one endogenous coding sequence, which codes for an HCP being difficult-to-remove (drHCP).

**Table 5** below summarizes a list of drHCPs, for which the mean relative concentration, named "MRC" in **Table 5** below, was determined as detailed above for **Table 4.** As already defined above, a drHCP is one that is present at least after a protein A column purification (ProA), after virus inactivation (VI), after cation exchange chromatography (CEX), or even after anion exchange chromatography (AEX), when PrA, VI, CEX and AEX are conducted as a series of subsequent purification steps.

Therefore, modifying at least a drHCP, preferably at the same time being an acHCP, or modifying a mix of at least two drHCPs and acHCPs, or both, can significantly reduce the amount of HCPs in a targeted way by specifically defining a set of acHCPs and/or drHCPs in a first step to select and modify the right panel of candidates.

**Table 5**

| Protein name | Uniprot ID | Location | MRC | ProA | VI | CEX | AEX |
|---|---|---|---|---|---|---|---|
| Thrombospondin-1 | G3HHV4 | extracellular | 6,54% | x | x | | |
| Fibronectin | G3I1V3 A0A3L7IDB0 | extracellular | 4,53% | x | x | | |
| Pyruvate kinase | G3H3Q1 | intracellular | 2,68% | x | x | | |
| Clusterin | G3HNJ3 | extracellular | 2,49% | x | x | x | |
| Peroxiredoxin-1 | G3GYP9 | intracellular | 2,36% | x | x | x | x |
| Glyceraldehyde-3-phosphate dehydrogenase | P17244 G3HMD1 | intracellular | 2,14% | x | x | x | |
| Actin beta | G3GVD0 | intracellular | 1,85% | x | x | x | |
| Sulfated glycoprotein 1 | G3I1Y9 | extracellular | 1,83% | x | x | x | |
| L-lactate dehydrogenase | G3I255 | intracellular | 1,55% | x | | | |
| Basement membrane-specific heparan sulfate proteoglycan core protein | G3HIM1 A0A3L7I8L8 | plasma membrane | 1,54% | x | | | |
| Phosphopyruvate hydratase | G3IAQ0 | intracellular | 1,46% | x | x | | |
| Lipoprotein lipase | G3H6V7 A0A3L7IKX6 | extracellular | 1,31% | x | | | |
| Endoplasmic reticulum chaperone BiP | G3I8R9 | intracellular | 1,25% | x | x | x | |
| Heat shock cognate 71 kDa protein | P19378 | intracellular | 1,24% | x | | | |
| Elongation factor 2 | G3HSL4 P09445 | intracellular | 1,16% | x | x | | |
| Cathepsin B | G3H0L9 | intracellular | 1,15% | x | x | x | |
| Heat shock protein HSP 90-alpha | P46633 | intracellular | 1,11% | x | | | |
| Glutathione S-transferase | G3I3Y6 | intracellular | 1,00% | x | x | x | |
| Elongation factor 1-alpha 1 | P62629 G3HH39 | intracellular | 0,96% | x | | | |
| Nidogen-1 | G3I3U5 | extracellular | 0,84% | x | | | |
| Ubiquitin | G3I129 | intracellular | 0,80% | x | x | x | x |
| Serine protease HTRA1 | G3IBF4 | extracellular | 0,78% | x | x | | |
| Carboxypeptidase | G3H8V5 | extracellular | 0,66% | x | x | x | |
| Peptidyl-prolyl cis-trans isomerase | G3HIQ1 | intracellular | 0,66% | x | | | |
| Endoplasmin | G3HQM6 | intracellular | 0,58% | x | x | | |
| Phosphoglycerate kinase 1 | P50310 | intracellular | 0,56% | x | | | |
| Histone H2B | G3INX0 | intracellular | 0,49% | x | | | |
| Tubulointerstitial nephritis antigen-like | G3H1W4 | intracellular | 0,47% | x | x | | |
| Annexin | G3IG05 | extracellular | 0,38% | x | x | x | x |
| Histone H2A type 1 | G3HDT6 | intracellular | 0,37% | x | | | |
| Calreticulin | G3HCX8 | intracellular | 0,36% | x | x | | |
| Galectin | G3I4Z7 A0A3L7I2M5 | extracellular | 0,36% | x | | | |
| Osteopontin | G3IKQ9 | extracellular | 0,27% | x | x | x | |
| 6-phosphogluconate dehydrogenase, decarboxylating | G3IHY5 | intracellular | 0,23% | x | | | |
| Protein S100 | G3HC31 | intracellular | 0,21% | x | x | | |
| 60S acidic ribosomal protein P0 | G3GU76 G3HKG9 | intracellular | 0,21% | x | | | |
| Malate dehydrogenase | G3HDQ2 | intracellular | 0,20% | x | | | |
| Granulins | G3HLK3 | extracellular | 0,20% | x | | | |
| Protein disulfide-isomerase | G3H0U6 | intracellular | 0,14% | x | | | |
| GTP-binding nuclear protein Ran | G3IHE5 | intracellular | 0,14% | x | | | |
| 14-3-3 protein theta | G3IEY9 | intracellular | 0,13% | x | | | |
| Hypoxia up-regulated protein 1 | G3I973 | intracellular | 0,06% | x | | | |
| Elongation factor 1-gamma | G3IE48 | intracellular | 0,02% | x | | | |
| Histone H2A | G3HDS3 | intracellular | <LLOQ | x | | | |
| Histone H4 | G3HPV6 | intracellular | <LLOQ | x | | | |
| 40S ribosomal protein SA | G3HQX0 | intracellular | <LLOQ | x | | | |
| Tubulin alpha chain | G3I6I6 | intracellular | <LLOQ | x | | | |
| Peptidyl-prolyl cis-trans isomerase A | G3IED5 | intracellular | <LLOQ | x | x | | |
| Tubulin beta chain | G3IEG6 | intracellular | <LLOQ | x | | | |

In certain embodiments, it may be preferable to modify more than one acHCP and/or drHCP in one and the same setting in a multiplexing approach. First, this allows for the reliable elimination of certain abundant and difficult-to-remove HCPs in the final biopharmaceutical independent of any upstream processing methods or downstream processing methods whilst simultaneously maintain a high performance rate of the CHO cell regarding its growth and titer capacity to produce high yields of a functional therapeutic agent of interest. Secondly, performing the knock-out in one and the same assay reduces the number of iterative modifications to a host cell to be modified, when multiple HCPs are targeted in one and the same experiment. In general, all target HCPs specified in any one of Tables 1 to 3 above, alone or in combination, qualify as suitable knock-out target.

In a specific embodiments, at least one, at least 2, at least three, at least four, at least five, at least sic, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty seven, at least twenty-eight, at least twenty-nine, at least thirty, or more, or all HCP targets according to Table 1, 2, or 3 above may be simultaneously knocked-out to obtain a modified cell according to the present disclosure. This is due to the fact that it could be shown experimentally that the impact of single knock-outs of exemplary HCPs taken from Table 1 and Table 2 on growth/viability and productivity/titer of the engineered cells has been evaluated under bioprocess conditions by generating stable mAb expressing cell clones followed by fed-batch runs in shake flask and was not negative so that the targets are suitable for knock-outs alone or in combination.

A matrix showing preferred combinations of HCP targets suitable for a multiple knock-down and/or knock-out is shown in **Figure 5****.** Particularly, as detailed in **Example 5** for certain target, multiple knock-down and/ or knock-outs of HCPs including at least one of, or a specific combination of FN, LPL, THBS, BGN, NID-1, PCOLCE, PXDN, THBS, PKM, CSPG4, LDHA, VIM, HSPA5, PPIB, HSP90AA1, LAMA5, FSTL1, and/or AEBP1 or any ortholog, homolog or paralog thereof, and/or additionally including at least one of PSAP, PRDX, HSPG2, CTSB, LGALS3BP, TNX, LAMB1, ITIH5, or TIMP.

In preferred embodiments of combined knock-outs, at least one of the knock-out targets is FN, LPL, or THBS. As evident from **Tables 4** and **5** above, the present inventors were able to provide a reliable scheme for categorizing HCPs under the plethora of proteins present in a host cell, by defining subsets of acHCPs and drHCPs under relevant settings, which selection scheme as such serves as a basis to define a knock-out or modification panel aiming at removing more than one HCP from a host cell in a convenient way. In certain embodiments of combined knock-outs, at least two of the knock-out targets are selected from FN, LPL, and THBS. In yet further embodiments of combined knock-outs, at least the three knock-out targets are selected from FN, LPL, and THBS, wherein further targets, particularly as defined above in any one of Tables 1, 2, and 3, and preferably of Tables 4 and 5 are combined with the FN, LPL, THBS knock-out panel.

In one embodiment, the at least one modification may thus be present in at least one endogenous coding sequence, which codes for Thrombospondin-1 (Uniprot ID: G3HHV4; SEQ ID NO: 1).

In another embodiment, the at least one modification may be present in at least one endogenous coding sequence, which codes for Fibronectin (Uniprot ID: G3I1V3; SEQ ID NO: 2).

In a yet another embodiment, the at least one modification may be present in at least one endogenous coding sequence, which codes for Sulfated glycoprotein 1 (Uniprot ID: G3I1Y9; SEQ ID NO: 9).

In yet another embodiment of the present invention, the at least one modification may be present in at least one endogenous coding sequence, which codes for Chondroitin sulfate proteoglycan 4 (=CSPG4) (Uniprot ID: G3H0E4; SEQ ID NO: 10).

In one embodiment of the present invention, the modified CHO cell may be derived from CHO-DG44 and the at least one modification may be present in at least one endogenous coding sequence, which codes for Thrombospondin-1 (Uniprot ID: G3HHV4; SEQ ID NO: 1).

In yet a further embodiment of the present invention, the modified CHO cell may be derived from CHO-DG44 and the at least one modification may be present in at least one endogenous coding sequence, which codes for Fibronectin (Uniprot ID: G3I1V3; SEQ ID NO: 2).

In one embodiment of the present invention, the modified CHO cell may be derived from CHO-DG44 and the at least one modification may be present in at least one endogenous coding sequence, which codes for Sulfated glycoprotein 1 (Uniprot ID: G3I1Y9; SEQ ID NO: 9).

In one embodiment of the present invention, the modified CHO cell may be derived from CHO-K1 and the at least one modification may be present in at least one endogenous coding sequence, which codes for Chondroitin sulfate proteoglycan 4 (=CSPG4) (Uniprot ID: G3H0E4; SEQ ID NO: 10).

In another embodiment of the present invention, the modified CHO cell may be derived from CHO-K1 and the at least one modification may be present in at least one endogenous coding sequence, which codes for Thrombospondin-1 (Uniprot ID: G3HHV4; SEQ ID NO: 1).

In yet another preferred embodiment of the present invention, the modified CHO cell may be derived from CHO-K1 and the at least one modification may be present in at least one endogenous coding sequence, which codes for Fibronectin (Uniprot ID: G3I1V3; SEQ ID NO: 2).

In another embodiment of the present invention, the modified CHO cell may be derived from CHO-K1 and the at least one modification may be present in at least one endogenous coding sequence, which codes for Sulfated glycoprotein 1 (Uniprot ID: G3I1Y9; SEQ ID NO: 9).

In a further embodiment of the present invention, the modified CHO cell may be derived from CHO-K1 and the at least one modification may be present in at least one endogenous coding sequence, which codes for Chondroitin sulfate proteoglycan 4 (=CSPG4) (Uniprot ID: G3H0E4; SEQ ID NO: 10).

In yet a further embodiment of the present invention, the modified CHO cell may be derived from CHO-S and the at least one modification may be present in at least one endogenous coding sequence, which codes for Thrombospondin-1 (Uniprot ID: G3HHV4; SEQ ID NO: 1).

In one embodiment of the present invention, the modified CHO cell may be derived from CHO-S and the at least one modification may be present in at least one endogenous coding sequence, which codes for Fibronectin (Uniprot ID: G3I1V3; SEQ ID NO: 2).

In another embodiment of the present invention, the modified CHO cell may be derived from CHO-S and the at least one modification may be present in at least one endogenous coding sequence, which codes for Sulfated glycoprotein 1 (Uniprot ID: G3I1Y9; SEQ ID NO: 9).

In yet another embodiment of the present invention, the modified CHO cell may be derived from CHO-S and the at least one modification may be present in at least one endogenous coding sequence, which codes for Chondroitin sulfate proteoglycan 4 (=CSPG4) (Uniprot ID: G3H0E4; SEQ ID NO: 10).

In yet a further embodiment of the present invention, the modified CHO cell may be derived from CHO-DXB11 (synonymously referred to as CHO-DUKX) and the at least one modification may be present in at least one endogenous coding sequence, which codes for Thrombospondin-1 (Uniprot ID: G3HHV4; SEQ ID NO: 1).

In another embodiment of the present invention, the modified CHO cell may be derived from CHO-DXB11 (synonymously referred to as CHO-DUKX) and the at least one modification may be present in at least one endogenous coding sequence, which codes for Fibronectin (Uniprot ID: G3I1V3; SEQ ID NO: 2).

In yet a further embodiment of the present invention, the modified CHO cell may be derived from CHO-DXB11 (synonymously referred to as CHO-DUKX) and the at least one modification may be present in at least one endogenous coding sequence, which codes for Sulfated glycoprotein 1 (Uniprot ID: G3I1Y9; SEQ ID NO: 9).

In another embodiment of the present invention, the modified CHO cell may be derived from CHO-DXB11 (synonymously referred to as CHO-DUKX) and the at least one modification may be present in at least one endogenous coding sequence, which codes for Chondroitin sulfate proteoglycan 4 (=CSPG4) (Uniprot ID: G3H0E4; SEQ ID NO: 10).

In yet another embodiment of the present invention, the modified CHO cell may be derived from CHO-Pro minus and the at least one modification may be present in at least one endogenous coding sequence, which codes for Thrombospondin-1 (Uniprot ID: G3HHV4; SEQ ID NO: 1).

In another embodiment of the present invention, the modified CHO cell may be derived from CHO-Pro minus and the at least one modification may be present in at least one endogenous coding sequence, which codes for Fibronectin (Uniprot ID: G3I1V3; SEQ ID NO: 2).

In a preferred embodiment of the present invention, the modified CHO cell may be derived from CHO-Pro minus and the at least one modification may be present in at least one endogenous coding sequence, which codes for Sulfated glycoprotein 1 (Uniprot ID: G3I1Y9; SEQ ID NO: 9).

In a preferred embodiment of the present invention, the modified CHO cell may be derived from CHO-Pro minus and the at least one modification may be present in at least one endogenous coding sequence, which codes for Chondroitin sulfate proteoglycan 4 (=CSPG4) (Uniprot ID: G3H0E4; SEQ ID NO: 10).

In yet another embodiment of the present invention, the modified CHO cell may be derived from a CHO GS cell and the at least one modification may be present in at least one endogenous coding sequence, which codes for Thrombospondin-1 (Uniprot ID: G3HHV4; SEQ ID NO: 1) or any homologue, orthologue, or paralogue thereof, and/or an HCP individually selected from SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, or any homologue, orthologue, or paralogue thereof, or any combination of these HCPs with each other, or with further HCPs.

In yet another embodiment of the present invention, the modified CHO cell may be derived from a CHO GS cell and the at least one modification may be present in at least one endogenous coding sequence, which codes for Fibronectin (Uniprot ID: G3I1V3; SEQ ID NO: 2) or any homologue, orthologue, or paralogue thereof, and/or an HCP individually selected from SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, or any homologue, orthologue, or paralogue thereof, or any combination of these HCPs with each other, or with further HCPs.

In yet another embodiment of the present invention, the modified CHO cell may be derived from a CHO GS cell and the at least one modification may be present in at least one endogenous coding sequence, which codes for Sulfated glycoprotein 1 (Uniprot ID: G3I1Y9; SEQ ID NO: 9) or any homologue, orthologue, or paralogue thereof, and/or an HCP individually selected from SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, or any homologue, orthologue, or paralogue thereof, or any combination of these HCPs with each other, or with further HCPs.

In yet another embodiment of the present invention, the modified CHO cell may be derived from a CHO GS cell and the at least one modification may be present in at least one endogenous coding sequence, which codes for Chondroitin sulfate proteoglycan 4 (=CSPG4) (Uniprot ID: G3H0E4; SEQ ID NO: 10) or any homologue, orthologue, or paralogue thereof, and/or an HCP individually selected from SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, or any homologue, orthologue, or paralogue thereof, or any combination of these HCPs with each other, or with further HCPs.

In a preferred embodiment, the modified CHO cell according to the present invention may contain one or more exogenously added gene(s) coding for one or more recombinant molecules, including at least one recombinant protein or peptide, RNA or DNA of interest. Preferably, said one or more recombinant molecule is a recombinant protein or peptide of interest representing a biopharmaceutical protein being selected from the exemplary group consisting of a recombinant protein or peptide, including monoclonal antibodies or a single-chain Fv, or any other antibody-format, including mono-, bi- and multispecific antibodies, chimeric antigen receptors, virus-like proteins, vaccines, and immunogenic compounds of any kind.

In one embodiment of the present invention, the modified CHO cell may be derived from CHO-DG44 and the at least one modification may be present in at least one endogenous coding sequence, which codes for a protein selected from group consisting of Thrombospondin-1 (Uniprot ID: G3HHV4; SEQ ID NO: 1), Fibronectin (Uniprot ID: G3I1V3; SEQ ID NO: 2), Sulfated glycoprotein 1 (Uniprot ID: G3I1Y9; SEQ ID NO: 9)), and Chondroitin sulfate proteoglycan 4 (=CSPG4) (Uniprot ID: G3H0E4; SEQ ID NO: 10) and the modified CHO cell may contain one exogenously added gene coding for a recombinant protein of interest, wherein said recombinant protein of interest is a monoclonal antibody, or a fragment or domain thereof, or any further therapeutic protein, including a bispecific antibody, fusion proteins, peptibodies, and peptides, or any prophylactic agent, including a vaccine, or a fragment or portion thereof.

In one embodiment, at least one of the protein to be knocked-out is selected from at least one of, or a specific combination of a knock-out of the HCP(s) FN, LPL, THBS, BGN, NID-1, PCOLCE, PXDN, THBS, PKM, CSPG4, LDHA, VIM, HSPA5, PPIB, HSP90AA1, LAMA5, FSTL1, and/or AEBP1 or any ortholog, homolog or paralog thereof, and/or additionally including at least one of PSAP, PRDX HSPG2, CTSB, LGALS3BP, TNX, LAMB1, ITIH5, or TIMP.

In a preferred embodiment of all aspects and embodiments as disclosed herein, at least one of FN, LPL, THBS, BGN, NID-1, PCOLCE, PXDN, THBS, PKM, CSPG4, LDHA, VIM, HSPA5, PPIB, HSP90AA1, LAMA5, FSTL1, AEBP1, PSAP, PRDX HSPG2, CTSB, LGALS3BP, TNX, LAMB1, ITIH5, and/or TIMP, or any ortholog, homolog or paralog thereof is knock-out.

In one embodiment of all aspects as disclosed herein, the modified CHO cell may be derived from CHO-DG44 and the at least one modification may be present in at least one endogenous coding sequence, which codes for a protein selected from group consisting of at least one, or two, or more protein(s) being both classified as a drHCP and as an acHCP, or wherein said at least one endogenous protein has an amino acid sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, respectively, including, for example, at least one of Thrombospondin-1 (Uniprot ID: G3HHV4; SEQ ID NO: 1), Fibronectin (Uniprot ID: G3I1V3; SEQ ID NO: 2), Sulfated glycoprotein 1 (Uniprot ID: G3I1Y9; SEQ ID NO: 9), and Chondroitin sulfate proteoglycan 4 (=CSPG4) (Uniprot ID: G3H0E4; SEQ ID NO: 10) and the modified CHO cell may contain more than one, preferably two, three, four, or five, exogenously added genes coding for recombinant proteins of interest.

In another embodiment of all aspects as disclosed herein, the modified CHO cell may be derived from CHO-DG44 and the at least one modification may be present in at least one endogenous coding sequence selected from the group consisting of at least one, or two, or more protein(s) being both classified as a drHCP and as an acHCP, or wherein said at least one endogenous protein has an amino acid sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, respectively, including, for example, at least one of which codes for Thrombospondin-1 (Uniprot ID: G3HHV4; SEQ ID NO: 1), Fibronectin (Uniprot ID: G3I1V3; SEQ ID NO: 2), Sulfated glycoprotein 1 (Uniprot ID: G3I1Y9; SEQ ID NO: 9), and/or Chondroitin sulfate proteoglycan 4 (=CSPG4) (Uniprot ID: G3H0E4; SEQ ID NO: 10), or any other HCP, preferentially as listed in Table 3, 4, and 5 or any homologue, orthologue, or paralogue thereof, and the modified CHO cell may contain one exogenously added gene coding for a recombinant protein of interest.

In yet another embodiment of all aspects as disclosed herein, the modified CHO cell may be derived from CHO-K1 and the at least one modification may be present in at least one endogenous coding sequence selected from the group consisting of at least one, or two, or more protein(s) being both classified as a drHCP and as an acHCP, or wherein said at least one endogenous protein has an amino acid sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, respectively, including, for example, at least one of which codes for Thrombospondin-1 (Uniprot ID: G3HHV4; SEQ ID NO: 1), Fibronectin (Uniprot ID: G3I1V3; SEQ ID NO: 2), Sulfated glycoprotein 1 (Uniprot ID: G3I1Y9; SEQ ID NO: 9), and/or Chondroitin sulfate proteoglycan 4 (=CSPG4) (Uniprot ID: G3H0E4; SEQ ID NO: 10), or any other HCP, preferentially as listed in Table 3, 4, and 5 or any homologue, orthologue, or paralogue thereof, and the modified CHO cell may contain one exogenously added gene coding for a recombinant protein of interest.

In yet a further embodiment of all aspects as disclosed herein, the modified CHO cell may be derived from CHO-K1 and the at least one modification may, for example, be present in at least one endogenous coding sequence, which codes for Fibronectin (Uniprot ID: G3I1V3; SEQ ID NO: 2) and the modified CHO cell may contain one exogenously added gene coding for a recombinant protein of interest, wherein said recombinant protein of interest is a monoclonal antibody, or a fragment or domain thereof.

In yet another embodiment of all aspects of the present disclosure, the modified CHO cell may be derived from CHO-S and the at least one modification may be present in at least one endogenous coding sequence selected from the group consisting of at least one, or two, or more protein(s) being both classified as a drHCP and as an acHCP, orwherein said at least one endogenous protein has an amino acid sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, respectively, including, for example, at least one of which codes for Thrombospondin-1 (Uniprot ID: G3HHV4; SEQ ID NO: 1), Fibronectin (Uniprot ID: G3I1V3; SEQ ID NO: 2), Sulfated glycoprotein 1 (Uniprot ID: G3I1Y9; SEQ ID NO: 9), and/or Chondroitin sulfate proteoglycan 4 (=CSPG4) (Uniprot ID: G3H0E4; SEQ ID NO: 10), or any other HCP, preferentially as listed in Table 3, 4, and 5 and the modified CHO cell may contain one exogenously added gene coding for a recombinant protein of interest.

In a preferred embodiment, the modified CHO cell may be derived from CHO-S and the at least one modification may be present in at least one endogenous coding sequence, which codes for a protein selected from the group, for example, consisting of Thrombospondin-1 (Uniprot ID: G3HHV4; SEQ ID NO: 1), Fibronectin (Uniprot ID: G3I1V3; SEQ ID NO: 2), Sulfated glycoprotein 1 (Uniprot ID: G3I1Y9; SEQ ID NO: 9), Chondroitin sulfate proteoglycan 4 (=CSPG4) (Uniprot ID: G3H0E4; SEQ ID NO: 10) and the modified CHO cell may contain more than one, preferably two, three, four, or five, exogenously added genes coding for recombinant proteins of interest, wherein at least one, preferably two, three, four, five, or all, of said recombinant proteins of interest are monoclonal antibodies, or a fragment or domain thereof.

In yet a further embodiment of all aspects of the present invention, the modified CHO cell may be derived from CHO-DXB11 (synonymously referred to as CHO-DUKX) and the at least one modification may be present in at least one endogenous coding sequence selected from the group consisting of at least one, or two, or more protein(s) being both classified as a drHCP and as an acHCP, or wherein said at least one endogenous protein has an amino acid sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, respectively, including, for example, at least one of which codes for Thrombospondin-1 (Uniprot ID: G3HHV4; SEQ ID NO: 1), Fibronectin (Uniprot ID: G3I1V3; SEQ ID NO: 2), Sulfated glycoprotein 1 (Uniprot ID: G3I1Y9; SEQ ID NO: 9), and/or Chondroitin sulfate proteoglycan 4 (=CSPG4) (Uniprot ID: G3H0E4; SEQ ID NO: 10), or any other HCP, preferentially as listed in Table 3, 4, and 5 and the modified CHO cell may contain one exogenously added gene coding for a recombinant protein of interest.

In a preferred embodiment of the present invention, the modified CHO cell may be derived from CHO-DXB11 (synonymously referred to as CHO-DUKX) and the at least one modification may be present in at least one endogenous coding sequence, which codes for a protein selected from group, for example, consisting of Thrombospondin-1 (Uniprot ID: G3HHV4; SEQ ID NO: 1), Fibronectin (Uniprot ID: G3I1V3; SEQ ID NO: 2), Sulfated glycoprotein 1 (Uniprot ID: G3I1Y9; SEQ ID NO: 9), and Chondroitin sulfate proteoglycan 4 (=CSPG4) (Uniprot ID: G3H0E4; SEQ ID NO: 10) and the modified CHO cell may contain more than one, preferably two, three, four, or five, exogenously added genes coding for recombinant proteins of interest, wherein at least one, preferably two, three, four, five, or all, of said recombinant proteins of interest are monoclonal antibodies, or a fragment or domain thereof.

In a particularly preferred embodiment of the present invention, the modified CHO cell may be characterized by a reduced load of at least Fibronectin (Uniprot ID: G3I1V3; SEQ ID NO: 2) and at least one, preferably at least two, preferably at least three, preferably at least four, preferably at least five, preferably at least six, preferably at least seven, preferably at least eight, preferably at least nine, preferably at least ten, preferably at least eleven, preferably at least twelve, preferably at least thirteen, preferably at least fourteen, preferably at least fifteen, preferably at least sixteen, preferably at least seventeen, preferably at least eighteen, preferably at least nineteen, other endogenous protein(s) independently selected from the group consisting of the proteins according to SEQ ID NO: 1, 4, 5, 6, 7, 8, 9, 11, 12, 13, 127, 14, 16, 17, 19, 20, 21, 22, 23, 24, 27, 31, 34, 35, 41, 49, and 55 as listed in Table 3, 4 and/or 5 or any homologue, orthologue, or paralogue thereof.

In a preferred embodiment of the CHO cell according to the present invention, the at least one modification leads to the reduced transcription and/or reduced functional expression of said endogenous protein(s) thereby lowering the total content of drHCPs and/or acHCPs.

In one embodiment of the present invention, the at least one modification may lead to premature transcription termination resulting in a truncated and thus inactive form of the respective protein. Preferably, the at least one modification may lead to premature transcription termination in exon 1 of the respective protein. In another preferred embodiment, the mutation may be in another exon than exon 3, e.g., in case another exon is easier accessible for successful and efficient editing.

Preferably, premature transcription termination may occur in the first 50%, preferably in the first 40%, preferably in the first 30%, preferably in the first 20%, preferably in the first 10%, preferably in the first 5%, preferably in the first 2% of the respective genomic coding sequence viewed from its transcriptional start, i.e. its start codon.

By prematurely terminating the transcription of a given target gene as close to its respective transcriptional start as possible the amount of formed transcript is kept to a minimum and the overall load of the respective protein in the modified CHO cell according to the present invention is minimized. Thereby, the positive effect in terms of an optimized downstream processing efficiency is maximized.

Preferably, the CHO cell according to the present invention comprises at least one recombinant gene encoding at least one recombinant protein of interest and/or encoding at least one recombinant RNA molecule of interest.

In a preferred embodiment of all aspects and embodiments as disclosed herein, the at least one recombinant protein of interest may be selected from the group consisting of therapeutic proteins, monoclonal antibodies, bispecific antibodies, fusion proteins, peptibodies, and peptides.

In one embodiment of all aspects and embodiments as disclosed herein, the at least one recombinant RNA molecule of interest may be selected from the group consisting of mRNA therapeutics including mRNA vaccines, non-coding single-stranded RNA species, such as e.g. antisense RNAs and MicroRNAs (also called miRs), interfering RNAs (RNAi), such as e.g. small interfering RNA (siRNA) or micro RNA (miRNA), and RNA aptamers.

In a preferred embodiment of the CHO cell according to the present invention, the at least one recombinant protein of interest is a therapeutic molecule.

In a preferred embodiment of the CHO cell according to the present invention, the at least one modification is selected from the group consisting of at least one insertion, at least one deletions, and at least one substitution, including a base edit, or any combination thereof, preferably wherein said at least one modification is present in exon 1 of the respective endogenous coding sequence, and/or wherein said at least one modification in said coding sequence is a frame-shift mutation or a point mutation, the point mutation resulting in a stop codon.

In a preferred embodiment of the CHO cell according to the present invention, the at least one modification in said endogenous coding sequence(s) allows for an optimized downstream processing and/or guarantees a reduced load of total HCPs, (i) wherein the CHO cell comprises at least one recombinant non-endogenous gene encoding at least one recombinant protein or RNA of interest and wherein said optimized downstream processing is characterized by a reduced load of total HCPs in the recombinant protein or RNA of product of interest, wherein the total load of HCPs is reduced by at least 0.1%, at least 0.2%, at least 0.3%, at least 0.4%, at least 0.5%, at least 0.6%, at least 0.7%, at least 0.8%, at least 0.9%, preferably at least 1%, at least 1.25%, at least 1.5%, at least 1.75%, at least 2%, at least 2.25%, at least 2.5%, at least 2.75%, at least 3%, at least 3.25%, at least 3.5%, at least 3.75%, at least 4%, at least 4.5%, more preferably at least 5%, at least 5.5, at least 6%, at least 6.5%, at least 7%, at least 7.5%, at least 8%, and even more preferably at least 9%, at least 10%, at least 15%, at least 20%, at least 25%, at least 30%, at least 35%, at least 40%, at least 45%, or at least 50%, at least 55%, at least 60%, at least 65%, at least 70%, or at least 75% as determined by measuring the relative concentration of total HCPs in ng/mL per sample obtained in a modified CHO cell in comparison to a reference sample obtained from a CHO wild-type cell, and/or (ii) wherein said at least one modification in said endogenous coding sequence(s) leads to an improved upstream processing performance, wherein said improved upstream processing performance is characterized by an increased concentration of viable cells and/or an increased specific productivity and/or an increased final titer and/or an increased process duration in comparison to a wild-type cell not carrying the at least one modification in its genome.

In one preferred embodiment, total load of drHCPs is reduced by at least 0.1%, at least 0.2%, at least 0.3%, at least 0.4%, at least 0.5%, at least 0.6%, at least 0.7%, at least 0.8%, at least 0.9%, preferably at least 1%, at least 1.25%, at least 1.5%, at least 1.75%, at least 2%, at least 2.25%, at least 2.5%, at least 2.75%, at least 3%, at least 3.25%, at least 3.5%, at least 3.75%, at least 4%, at least 4.5%, more preferably at least 5%, at least 5.5, at least 6%, at least 6.5%, at least 7%, at least 7.5%, at least 8%, and even more preferably at least 9%, at least 10%, at least 15%, at least 20%, at least 25%, at least 30%, at least 35%, at least 40%, at least 45%, or at least 50%, at least 55%, at least 60%, at least 65%, at least 70%, at least 75%, at least 80%, at least 85%, at least 90%, at least 95%, or at least 99% as determined by measuring the relative concentration of total drHCPs in ng/mL per sample obtained in a modified CHO cell in comparison to a reference sample obtained from a CHO wild-type cell. Particularly for drHCPs, an increase of the amount of drHCPs removed, preferably above a certain threshold, is advantageous, as this class of drHCPs cannot be easily removed via conventional purification strategies so that impurities will remain in the in the biopharmaceuticals. This can be avoided by focusing on individual drHCPs, and preferably certain combinations thereof as defined herein to be knocked-out to significantly improve the purity of biopharmaceutical products obtained after manufacturing in a modified host cell of the present invention.

In a preferred embodiment of the present invention, the at least one modification in said endogenous coding sequence(s) may allow for an optimized downstream processing, wherein said optimized downstream processing is characterized by a reduced number of downstream processing steps required for obtaining a recombinantly produced molecule in its purified form as compared to the respective wild-type (i.e non-modified) CHO cell. For example, the at least one modification may have the advantage that at least one purification step, particularly an ion exchange chromatography step, i.e., anion or cation exchange chromatography, or even both, can be omitted, as the overall HCP load was increased that significantly by the methods of the present invention that these steps are no longer necessary to achieve the ultimate grade of purity. This represents a significant advantage, as additional column chromatography steps require resources, and each additional step leads to a decrease of the yield of a recombinant protein of interest to be purified.

Thus, the modified CHO cell according to the present invention allows for the downstream processing to be optimized in terms of cost efficiency, time efficiency, energy efficiency, and sustainability.

In certain embodiments related to the CHO cell according to the present invention, all alleles of said at least one endogenous coding sequence comprise at least one or more of said modification(s). In certain embodiments, only one allele may be targeted to achieve a favourable balance result between the reduction of the load of the respective HCP to be knocked-out in one allele and host cell integrity and stability in cases where a full knock-out might be detrimental to the cell, or might be at least detrimental in a sense that the cell growth, and recombinant protein production capacity is significantly reduced.

In a preferred embodiment of the CHO cell according to the present invention, the at least one endogenous protein has an amino acid sequence corresponding to any of the amino acid sequences according to SEQ ID NOs: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, and 128, or corresponds to a protein mentioned in any one of Table 1, 2, 3, 4 or 5 herein, or any homolog, ortholog or paralog thereof, or corresponding to an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to any one of the sequences according to SEQ ID NOs: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, and 128.

Preferably, the CHO cell according to the present invention comprises at least one modification in at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty or more different HCP encoding sequences, wherein each HCP is preferably selected from an acHCP and/or a drHCP.

In a further aspect, the present invention relates to a method for the production of at least one modified CHO cell according to the present invention comprising the steps: (i) providing at least one CHO cell comprising at least one endogenous target nucleic acid segment; (ii) providing at least one genome or transcriptome editing agent comprising at least one RNAi agent, or at least one editing agent comprising at least one site-directed endonuclease, preferably being selected from a meganuclease, a ZFN, a TALEN, a CRISPR-nuclease, or a nickase or nuclease-dead variant therefrom, or a nucleic acid molecule encoding the same , and optionally in case of a CRISPR-nuclease: providing at least one suitable, functional guide RNA molecule, or a nucleic acid molecule encoding the same; (iii) introducing into said at least one CHO cell the at least one genome editing agent of step (ii); (iv) obtaining at least one modified CHO cell comprising at least one modification in said at least one endogenous target nucleic acid segment according to step (i); (v) optionally: selecting a further target nucleic acid segment and repeating steps (i) to (iv) at least one time to obtain at least one modified CHO cell comprising at least one further modification in a further endogenous target nucleic acid segment.

In a preferred embodiment of the method according to the present invention, the at least one endogenous target nucleic acid segment may be a nucleic acid sequence, preferably a DNA sequence, coding for part of any of the amino acid sequences according to any of the SEQ ID NOs: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, and 128.

Preferably, said target nucleic acid segment may be a nucleic acid sequence, preferably a DNA sequence, coding for an amino acid sequence, which is contained in the first 50%, preferably in the first 40%, preferably in the first 30%, preferably in the first 20%, preferably in the first 10%, preferably in the first 5%, preferably in the first 2% of any of the amino acid sequences according to any of the SEQ ID NOs: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, and 128.

In a preferred embodiment of the method according to the present invention, a CRISPR/Cas system may be used as genome editing system. This clustered regularly interspaced short palindromic repeats (CRISPR) and CRISPR-associated protein (CRISPR/Cas) system has significantly altered molecular biology research and development over the last decade offering enormous possibilities to modify cells of interest, including complex eukaryotic cells, in a highly targeted way. Any CRISPR/Cas system suitable for eukaryotic cell genome editing can be used according to the present disclosure. The CRISPR/Cas system may include a CRISPR/Cas9 systems, a CRISPR/Cas13 systems, CRISPR/Cas12a system (also called CRISPR/Cas12a system), a CRISPR/C2C2 systems, CRISPR/CasX systems (also called CRISPR/Cas12e system), CRISPR/CasY systems, CRISPR/Cmr systems, CRISPR/Csm systems, CRISPR/MAD2 systems, CRISPR/MAD7 systems, CRISPR/CasZ systems, CRISPR/Cas14a, b or c systems, CRISPR/Cas phi systems, CRISPR/Cas12f systems, or catalytically active fragments or variants thereof. Various kinds of CRISPR systems and optimized mutated systems, including nickase or nuclease-dead based systems, CRISPR mutants with optimized PAM-specificities and temperature tolerance, and various methods of using these CRISPR variants and mutants alone or on combination with other effectors, e.g., to be suitable for base editing and prime editing, have been developed and are suitable to be used according the present disclosure (cf. Jinek, et al., A Programmable Dual-RNA-Guided DNA Endonuclease in Adaptive Bacterial Immunity. Science 2012, 337, 816-821; Hillary and Ceasar, Mol Biotechnol. 2022 Sep 27;1-15, doi: 10.1007/s12033-022-00567-0; Zetsche, et al. Cpf1 Is a Single RNA-Guided Endonuclease of a Class 2 CRISPR-Cas System. Cell 2015, 163, 759-771; Kim et al. Nat Biotechnol. (2022);40(1):94-102; doi: 10.1038/s41587-021-01009-z; Liu et al., CRISPR J. April 2020; 3(2): 97-108.; Karvalis et al. Nucleic Acids Res. (2020); 48(9):5016-5023. doi: 10.1093/nar/gkaa208, Selkova et al. RNA Biol. (2020); 17(10):1472-1479; doi: 10.1080/15476286.2020.1777378, Rees and Liu, 2018. Nat. Rev. Genet.; doi: 10.1038/s41576-018-0059-1, Komor et al., Nature 533, 420-460, 2016; Komor et al., 2017, Science Advances, doi:10.1126/sciadv.aao4774M; Gaudelli et al., Nature, 551: 464-471, 2017; Lu et al., nt. J. Mol. Sci. 2022, 23, 9862. https://doi.org/10.3390/ijms23179862).

In certain embodiments, CRISPR nucleases or variants thereof being suitable for multiplexing applications, meaning the targeting of more than one target site of interest in a genome to be modified in a site directed manner, may be preferred. CRISPR/Cas nucleases, or mutants or variants thereof, of the Class 2 Type V may be preferred in certain embodiments (cf. Koonin & Makarova. Mobile genetic elements and evolution of CRISPR-Cas systems: All the way there and back. Genome Biology and Evolution. 2017;9(10):2812-2825. doi: 10.1093/gbe/evx192, Tong et al., Front. Cell Dev. Biol 2021, Sec. Cellular Biochemistry doi.org/10.3389/fcell.2020.622103.

As it is known to one skilled in the art, CRISPR/Cas effectors need a guiding molecule to exert their activity. The "guide molecule", in particular the "guide RNA" (gRNA) may comprise a trans-activating CRISPR RNA (tracrRNA) and a CRISPR RNA (crRNA). Certain CRISPR nucleases, e.g., Cas12a, only require a crRNA. TracrRNA and crRNA can be combined in one molecule, resulting in a single guide RNA (sgRNA), which comprises the sequence information targeting the genomic sequence for cleavage by the nuclease. For multiplexing purposes, more than one gRNA allowing the recognition and thus modification of more than one target site in a genome may be designed and used in one and the same experiment, or one after another.

In a preferred embodiment of the method according to the present invention, the CRISPR-nuclease may be a Class 2 Type V nuclease, or a variant or mutant thereof, including a Cas12a (formerly known as Cpf1) or a MAD7 or a Cas14 effector. Suitable vectors and plasmids to express the relevant CRISPR effector and the cognate gRNA(s) are available to the skilled person and can be easily adapted by designing the gRNA(s) *in silico.*

In another embodiment, an RNAi agent can be used to knock-down or silence at least one RNA coding for at least one HCP target of interest. For RNAi, small RNAs function to guide specific effector proteins to a target nucleotide sequence by complementary base pairing resulting in degradation of the target. A "gene silencing construct" or "RNAi agent" usually comprises so called "sense" and "antisense" sequences. Sense and antisense sequences are complementary sequences, which are present in reverse orientation in a nucleic acid sequence. If a nucleic acid construct comprises a sense and a corresponding antisense sequence, the two complementary sequences form an RNA double strand upon transcription, which results in an "RNA hairpin". With these RNA hairpin sequences, more than one HCP target can be knocked-down simultaneously in certain embodiments.

In specific embodiments, a knock-down of at least one HCP via, for example, an RNAi agent and a knock-out mediated by genome editing, including CRISPR/Cas, may be combined to reduce the expression of at least one target HCP.

In an even further aspect of the invention, a method for the production of at least one recombinant protein of interest is provided comprising the steps: (a) providing at least one modified CHO cell according to the present invention, wherein the at least one modified CHO cell comprises at least one recombinant gene encoding at least one recombinant protein of interest; (b) culturing said at least one target cell in a culture medium such that at least one recombinant protein of interest is expressed; (c) harvesting said at least one recombinant protein of interest; (d) purifying and/ or decontaminating said at least one recombinant protein of interest.

In a preferred embodiment of the method according to the present invention, the at least one recombinant gene is exogenously expressed from at least one expression vector, i.e. at least one plasmid. Said expression vector may be particularly selected from the group consisting of mammalian expression vectors, as these are disclosed herein, or as these are known to one skilled in the art. The skilled person can thus easily define suitable expression vectors for a host cell of interest in his/her respective technical field that are compatible with the present disclosure.

In a preferred embodiment of the method according to the present invention, culturing said at least one target cell may be performed by a method selected from the group consisting of batch cultivation, fed-batch cultivation, perfusion cultivation, and the like, and combinations thereof. In a preferred embodiment of the method according to the present invention, the culture medium for culturing said at least one target cell may be selected from the group consisting of, for example, a non-chemically defined or a chemically-defined cell culture medium.

In one embodiment of the method according to the present invention, purifying and/or decontaminating may comprise one or more process steps selected from the group consisting of affinity purification, ion exchange, hydrophobic interaction chromatography, and size exclusion chromatography.

In a preferred embodiment of the method according to the present invention, the at least one recombinant protein of interest may be selected from the group consisting of, but not restricted to, therapeutic proteins, monoclonal antibodies, bispecific antibodies, fusion proteins, peptibodies, and peptides.

In a further aspect of the present invention, the use of at least one modified CHO cell according to any of the present invention for the production of at least one pharmaceutical drug, or a part thereof is provided. The pharmaceutical drug can be any biomolecule, preferably a recombinant molecule, including recombinant proteins, peptide, RNA, DNA, or any combination, fusion molecules, including covalently or non-covalently (e.g., hybridized; antigen::antibody complexes etc.) tethered molecules, that can be produced in a CHO cell of the present disclosure. The pharmaceutical drug will preferably be purified. Further, the pharmaceutical drug may be further modified, e.g., by chemical synthesis to attach an additional moiety, or to link two molecules to each other. Further, the pharmaceutical drug may be stabilized, lyophilized, and provided together with suitable buffers and agents all being pharmaceutically acceptable. Galenic techniques for compounding a pharmaceutical drug are known to one skilled in the art.

In a further aspect of the present invention, a kit is provided comprising (a) at least one modified CHO cell according to the present invention, the CHO cell or the kit comprising: (b.i) at least one nucleic acid molecule suitable for expressing at least one recombinant protein or peptide of interest; optionally means for introducing the same into the CHO cell genome, or alternatively (b.ii) at least one nucleic acid molecule encoding at least one recombinant protein of interest; optionally means for introducing the same into the CHO cell genome; or alternatively (b.iii) at least one nucleic acid molecule suitable for transcribing at least one recombinant RNA molecule of interest; optionally means for introducing the same into the CHO cell genome; or alternatively (b.iv) at least one nucleic acid molecule encoding at least one recombinant RNA molecule of interest; optionally means for introducing the same into the CHO cell genome; and optionally (c) culture medium suitable for the at least one modified CHO cell according to step (a) (i) to replicate the at least one nucleic acid molecule according to any of the steps (b.i), (b.ii), (b.iii), and (b.iv) endogenously or exogenously; and (ii.a) to express the at least one recombinant protein of interest according to step (b.i) and/or (b.ii), and/or to transcribe the at least one recombinant RNA molecule of interest according to step (b.iii) and/or (b.iv).

Further provided are methods for using the purified recombinant molecules, alone or in combination with further pharmaceutically acceptable ingredients and buffers, as produced by the modified CHO cells of the present invention in a method of treating a subject in need thereof. In view of the high degree of purity based on the reduction of the HCP amount, the recombinant molecules are particularly suitable to be safely applied to human beings.

In yet another aspect of the present disclosure, there is provided a method for identifying and optionally selecting a favourably modified host cell protein (HCP) profile of cell, preferably of a CHO cell, preferably a CHO cell characterized by a defined load of at least one endogenous protein being a difficult-to-remove HCP (drHCP) and/or an abundant core HCP (acHCP), wherein said method comprises: (i) providing at least one CHO cell, optionally a CHO cell expressing at least one recombinant molecule of interest; (ii) providing at least two, preferably at least 3, 4, 5, 6, 7, 8, 9, 10, or even more expression profiles for individual clones of at least one CHO cell expressing the same or a different recombinant molecule of interest; (iii) comparing and thus evaluating the data obtained from the expression profile analysis and correlating the data to at least one quantifiable parameter for each protein or RNA of interest; (iv) categorizing the data by attributing the values obtained for the at least one quantifiable parameter to at least one state or group; and (v) identifying favourably modified host cell protein (HCP) profile; the method optionally including preparing a cell, preferably a CHO cell, with a favourably modified HCP profile by modifying at least one endogenous HCP and optionally selecting and thus obtaining a modified cell, preferably a modified CHO cell, with a favourably modified HCP profile.

Correlation can be performed by in silico analytic methods. Usually, correlation will imply the alignment of the obtained data with publicly available genome, transcriptome and protein expression databases.

In one embodiment, the expression profile may be a transcriptome profile determining and/or quantifying RNA transcripts. In another embodiment, the expression profile may be a profile determining and/or quantifying a protein or peptide profile, preferably a high-throughput method including LC-MS and SWATH LC-MS.

In certain embodiments, the expression profile may be obtained by making a series of sample measurements over time, e.g., by taking and analysing a sample at certain time points before harvest, or by taking and analysing a sample after a post-harvest purification step, or by taking and analysing a sample, e.g., for a transcriptome profile, after stimulating a culture comprising the at least one CHO cell of interest, or a population thereof, with at least one stimulus or inducer to evaluate the influence of the stimulus or inducer (e.g., a chemical agent, a biological agent or any abiotic stress factor) on the cell or cell population. Modification of the at least one cell, preferably the at least one CHO cell, can be performed as defined herein for the various methods for providing a CHO cell with a modified HCP profile.

The above method thus integrate and thus facilitate high-throughput cell cultivation, screening and modification methodologies to expedite HCP target identification and to establish a pipeline for rapidly identifying HCP targets of interest for a setting of interest followed by a quick modification of the cell of interest, preferably in a multiplexing manner, to obtain optimized production CHO host cells in a short period of time.

In another embodiment there is provided a recombinant molecule as pharmaceutical agent, preferably a recombinant protein or peptide, including at least one monoclonal antibody, obtained from a modified CHO cell according to the present invention for use in a method of treating a subject in need thereof, including preventing a disease and curing a disease, wherein the treatment is associated with less side effects in view of the reduced HCP amount of the recombinant molecule as active pharmaceutical agent.

All individual embodiments and aspects as disclosed herein can be combined with each other within the framework and context of the present disclosure.

The present invention is now further illustrated by the following non-limiting examples.

### Examples

### Example 1: Generation of HCP profiles

To identify those HCPs to be knocked-out to obtain a CHO cell with an advantageously reduced HCP content, HCPs were first analyzed in a variety of samples in a systematic manner. To this end, the Uniprot Chinese hamster reference proteome was used as basis for an initial classification to identify the targets of outstanding interest.

HCP profiles were generated for a number of mAb-expressing CHO production clones that were processed under industrial USP (ambr250 fed-batch process) and DSP (standard ProA capture, virus inactivation [VI], cation exchange [CEX] and anion exchange [AEX] polishing steps) conditions. The different production clones, expressed antibodies, applied temperature and pH conditions as well as the sampling regime are shown in **Figure 1****.**

All samples were analyzed with SWATH LC-MS (for methodology, cf. Krasny et al., Journal of Proteomics Volume 189, 2018, Sim, et al., Sci Data 7, 263,2020) and for each sample the present HCPs were identified and quantified using the Uniprot Chinese hamster reference proteome.

### Example 2: Systematic identification of HCP knock-out

Based on the HCP profiles generated in Example 1, two groups of relevant HCP knock-out targets were identified:
(1) Abundant core HCPs (acHCPs), comprising those HCPs that were detected in all samples taken before or on the harvest day (labelled by the box "abundant HCPs" in **Fig. 1** and **Table 4)** and that were present at quantifiable amounts (> LLOQ) in all these samples.
(2) Difficult-to-remove HCPs, comprising those HCPs that were detected in at least one of the samples taken during the DSP processing (labelled by the box "difficult-to-remove HCPs" in **Fig. 1** and **Table 5).**

After each standard purification scheme using a cascade of purification steps was applied as detailed in Example 1, a sample was taken after each step and the sample was analyzed by SWATH LC-MS as well.

As a result of this extensive sample testing, a group of abundant core HCPs (cf. middle column in **Fig. 1**) and of difficult-to-remove HCPs (cf. right column in **Fig. 1**) was identified. The relevant HCP knock-out targets from the group of abundant core HCPs (= acHCPs) were defined based on the following criteria: (i) the HCPs are present in all samples labeled by the middle column in **Fig. 1****,** and (ii) the are present at quantifiable amounts (> LLOQ, cf. **Fig. 2**)**.** This information was then aligned with the UniProt Chinese Hamster reference genome.

**Figure 2** shows the identification process, classification and quantities of abundant core HCPs: in total, 1,254 different HCPs were detected in samples taken before or on harvest day, but only 351 of them were found in all these samples. 67 out of those were additionally present at precisely quantifiable amounts (> LLOQ) in all samples and were defined as abundant core HCPs **(acHCPs** see **Table 4** below). The identified abundant core HCP species were further characterized regarding their subcellular localization and measured average quantities: 39 HCPs were cytoplasmic proteins present at an average concentration of about 230 mg/L corresponding to about 32.6% of the total HCP amount, 23 HCPs were extracellular (average concentration about 163 mg/L, ca. 23.1% of total HCP) and 5 membrane-bound (27 mg/L, 3.8%) proteins, respectively.

According to the criteria defined above, 49 proteins were classified as difficult-to-remove HCPs **(drHCPs** see **Table 5** above).

Yielding an acHCP and a drHCP list was thus the result of a targeted screening process using bioinformatics and high-throughput analysis steps of a huge number of exemplary samples with the idea to obtain a guidance on relevant HCPs of major interest to be knocked-out. After analyzing (wet lab and in silico) a pool of 1.254 HCPs (present status, further work is ongoing), this object could be achieved by defining acHCPs and drHCPs as "core" HCPs of major importance in view of their abundance and/or inherent removal difficulty.

**Table 6** below summarizes the Uniprot IDs of all 1.254 HCPs currently identified and tested (work ongoing):

**Table 6**

| |
|---|
| G3H6V7, G3H0L9, G3HSX8, G3I1V3, G3GYP9, G3I1Y9, G3IKQ9, G3HHV4, G3HIM1, G3HWE4, G3HBI1, G3H3E4, G3I278, G3GR64, G3HZD1, G3HGW6, G3IBH0, G3I664, G3HMA1, G3HAI3, G3HPV7, G3H3Q1, G3HNJ3, G3H0E4, G3I255, G3HHR3, G3I8R9, P19378, G3H533, P46633, G3I3Y6, G3I3U5, G3GUU5, G3I129, G3IBF4, G3HKZ1, G3H8V5, G3HIQ1, G3IKC3, G3HQM6, G3HDS3, G3HH39, G3HKG9, G3HMD1, G3HPV6, G3HQX0, G3I6I6, G3IED5, G3IEG6, P09445, P38507, P17244, G3GVD0, G3H2T7, G3HHM2, G3IAQ0, G3HSL4, P14851, P62629, P62976, P50310, G3I1H5, G3I6T1, G3INX0, G3H1W4, G3H8V4, G3HBD4, G3H7I6, G3IF52, Q9EPP7, G3IG05, G3IBK2, G3HDT6, G3I4H6, G3HH30, G3HCX8, G3I4Z7, G3I4E8, P68361, G3IF80, G3H8F4, G3HLV6, G3HMV7, G3HC84, G3GXD7, G3HJY9, G3HQP8, A0A3L7HAT5, G3GU60, G3I5L3, G3HC29, Q8R4U2, G3HBG8, G3IIE7, G3IDM2, G3IHY5, G3IBF1, G3GTT2, G3ILK7, G3HC31, G3GU76, G3HG95, G3HDQ2, G3IKH9, G3I1V4, G3GXB0, G3HLK3, G3ICD3, G3HQL6, G3IEU2, Q9WV24, G3H1K9, G3HY08, G3I5A4, G3I3H2, G3I4W7, G3I073, G3GR73, G3H2J8, G3H935, G3HAN8, G3IPU3, G3H2I6, G3H1D5, G3HBD3, G3HN31, A0A3L7HQ14, G3HC64, A0A3L7I8J7, A0A3L7I8K8, G3I8B5, A0A8C2LJ19, A0A8C2N4D5, A0A8C2MC57, A0A8C2N0F8, G3GXY1, G3GXZ0, A0A8C2LRW3, G3HFS5, A0A8C2LYC0, A0A8C2QIZ6, A0A8C2QLM9, G3H996, A0A8C2MMI8, A0A8C2M328, A0A8C2M6G9, 055058, A0A8C2LVZ9, A0A8C2MY83, A0A8C2M0V0, G3IHE0, Q8K3H7, G3HW00, A0A8C2M0N5, G3HEY9, A0A8C2M1I7, A0A8C2MW41, G3H4H3, G3H4T5, G3HAP7, G3HG25, G3GYY6, G3HFM4, G3HB04, G3HJ89, G3I936, G3H0U6, G3HMI3, G3HFP1, G3HC01, G3IHE5, G3I877, G3HZD3, |
| G3IFJ6, G3HLS2, G3IFJ5, G3HG83, 035501, G3IEY9, G3I3G8, G3GZW8, G3IG23, G3IN86, G3I5Z5, G3HYJ8, G3HMG4, G3HYP6, G3HRK0, G3HPT8, G3H8C9, G3HY03, G3HN14, G3I5H3, G3IE21, P38982, G3GXZ0, G3IK05, G3HJM2, G3GZ85, G3IKN5, G3H6Y6, G3H7B3, G3IDN7, G3I4D4, G3I3Y7, G3HK00, G3H8N1, G3HZ42, G3H7K5, G3H928, G3HP89, G3GXT2, G3H7Z2, G3IH63, G3H705, G3IQ06, G3H2W6, G3IIB1, G3IDU7, G3INC5, O55044, G3HCW9, G3HU51, G3HGM6, G3GWR8, G3HGP6, G3IIK9, G3GRR8, G3H5V0, G3IKX2, G3HH34, G3I4N3, G3HQY6, G3HBP8, G3I5N6, G3I2M1, G3I3K5, G3H278, G3HM03, G3H352, G3GYZ1, G3I740, G3GSG4, G3I6W8, G3I0I7, G3IMD1, G3IF62, A0A061HTH1, G3HYJ9, P47961, G3I2E9, G3IDD4, G3I782, G3HSE4, G3IDE4, G3H354, G3IAS8, G3IBF7, G3HN88, G3H0C2, G3I351, G3GWQ1, G3I948, G3H092, G3I8S7, G3IBG3, G3ILF1, G3I9G7, G3GU30, G3HQD5, G3HKG8, G3HJD8, G3GZ90, G3GVX1, G3IHM2, G3GY75, G3GZZ0, G3H284, G3HA23, G3H577, G3HYB7, G3GZB2, G3HRQ4, G3IH84, G3I412, G3I692, G3INM2, G3H4I2, G3HUU6, G3I973, G3H2E2, G3GVX2, G3I2I9, G3IDD9, G3HMQ0, G3HKB0, G3HXL1, G3HKN1, G3GT05, G3H8K2, G3HEI6, G3GUV4, G3I5R2, G3HP75, G3HDD4, G3HZE9, G3HSF3, G3GUI3, G3GT45, G3I737, G3HXN7, G3INL9, G3I1R2, P50309, G3GWE9, G3HD97, G3H7M8, G3HKX2, G3H1M4, G3GT06, G3HV73, G3HSM3, G3HDR3, G3HWE7, G3I595, G3I2Y1, G3HWP9, G3H5D5, G3H4Z5, P00494, P47952, G3GYP7, 088818, G3HLT3, G3HES3, G3HMY0, G3IDT6, G3GT56, Q9JI55, G3I9X8, G3HNG2, G3H6Y5, G3H497, G3HZX6, G3H584, G3H6T5, G3HTG9, G3IHC7, G3HR08, G3I9S3, G3IKE2, G3IA10, Q9Z313, G3GYC6, G3HS88, G3H6Z4, G3HDU1, G3HBX0, P18687, G3GUV3, G3IBK8, G3H8W5, G3GW88, G3HC47, G3GTB7, G3H2K2, G3I064, G3IBL4, G3I979, G3GTX5, G3I1I8, Q9QXP3, G3HY23, G3ILN5, G3HDE5, G3HPE9, G3IIA1, G3HDL6, G3IB99, G3GY17, G3HZE3, G3HTE5, G3I7A8, G3II69, G3GYR9, G3IEF1, G3HSB4, G3IEG2, G3I9D3, G3HZW3, G3H3E6, G3HF46, G3IEE6, G3ILF3, G3HRM8, G3HDQ1, G3IMP5, G3GUD8, G3HIC5, G3GZ17, G3H4V1, G3HMP4, G3HNJ6, G3H018, G3I505, G3H3E3, G3IEB7, G3H6S8, G3H945, G3HE67, G3HCX3, G3HEQ3, G3H3C0, G3HQ45, G3HAF4, G3H4S3, G3H559, G3HU28, G3GYL5, G3HMV2, G3I881, G3H1U2, G3HZY1, P37880, G3H4A9, G3HTJ2, G3HEL2, G3GZR1, Q7SIA2, G3GZE6, Q9WVH0, G3HZV0, G3I151, G3H6C5, G3IC99, G3HRN0, G3H5W4, G3I004, G3H7R4, P26636, G3IEK1, G3I334, G3GX96, G3I3V6, G3HUI4, G3H289, G3HTV2, G3HD67, G3HI29, G3GWG3, G3GVB5, G3H604, G3HJT6, G3HNT9, G3IKQ6, G3H2A9, G3HIQ3, G3H8Y5, G3HR96, G3HD18, G3HW36, G3GS70, G3H7F9, G3I0F7, G3HNR3, G3I952, G3H0Q0, G3H893, G3I683, G3HFG4, G3H303, G3I5Q9, G3H609, G3H1B3, G3HRD3, G3I596, G3HRK9, P62265, G3IF39, G3H9V0, G3HR88, G3HP71, G3IHT7, G3IK13, G3H1Y8, G3IGI0, G3H0U9, G3H6K1, G3H894, G3H8Q4, G3HAQ2, G3H3G9, G3IMH4, G3HKD6, G3GX14, G3I3J3, G3HH02, |
| G3I8P7, G3HZF4, G3HMB9, G3H601, G3HNR0, G3H7J9, G3H8F9, G3HLD0, G3H1F4, G3HV18, G3IJJ0, G3INU6, G3GY47, G3I2H0, G3HG40, G3H0S4, G3IGL0, G3H638, G3I5S7, G3I072, G3H3P8, G3HNK4, G3HCV4, G3HIM4, G3IFK8, G3HMI0, G3ILP4, G3H4D3, P12269, G3GUX9, G3I7J6, G3IA72, G3H319, G3HSE5, G3HEZ1, G3GZD2, G3I2M2, G3HCH0, G3H8A8, G3I437, G3GWM0, G3GSB7, G3H7F8, G3IL75, G3IE48, G3IG96, Q9R152, G3HKY0, G3HUU7, G3I3W6, G3H2C4, G3HHH6, G3HCK9, G3IMV1, G3GXH4, G3IH46, G3HID6, G3I6J4, G3H1Z4, G3GUR1, G3H2P3, G3HK90, G3GVP7, G3HID1, G3IH79, G3IPS0, G3HG16, G3HXK5, G3IJV7, G3IPP8, G3H8N3, G3HBW5, G3H6U1, G3H6M5, G3II56, G3I2L7, G3HJS0, G3IA94, G3IJ85, G3I0Q1, G3H1V1, G3H412, G3HRD9, G3I196, G3H585, G3HJJ1, G3IHB0, G3H194, G3HJB0, G3HEE8, G3HGW7, G3HET8, G3HSH4, G3GR90, G3I0M2, G3H0F1, G3I2D3, G3I094, Q6E6J6, G3IGI6, G3HGP4, G3INF7, G3IDS2, G3H892, G3H1E5, G3H1G7, P05044, G3I1P5, G3H6E1, G3I6V9, G3HMS8, G3I784, G3IE04, G3HLU5, Q6LDZ8, G3GXU1, G3HJB1, G3I9A3, G3I4E6, G3GY95, G3HIY3, G3IN18, G3HYT5, G3I8H3, G3I638, G3HYH7, G3HAP1, G3GUP7, G3HNA5, G3IH96, G3HWP7, G3H453, G3H697, G3IAG0, G3HV39, G3H1N2, G3H5W5, Q8HDG8, G3I757, G3H9Z4, G3HAU1, G3HT18, G3HKQ7, G3IKQ5, G3I3X6, G3HA25, G3HPC9, G3IBI0, G3GSW3, G3HNV4, G3I0G1, G3HA54, G3GUY5, G3HE84, G3H9H7, G3HZP8, G3IAP3, G3I501, Q9Z2J2, G3I9W6, G3HC39, G3II47, G3GZL7, G3GY52, G3HKJ8, G3HRR5, G3HRJ5, G3HN77, G3GWQ3, G3I968, G3I9X6, G3I9Z3, G3HEY8, G3H0T1, G3I581, G3I027, G3HRM9, G3I6W5, G3HH92, G3H6B5, G3GWP1, G3I3H9, G3HMW7, G3H3X1, G3HW06, G3HIQ0, G3I5W3, G3HLZ2, G3H154, G3HW66, G3I4P0, G3HQB7, G3HMU4, G3HW48, G3GXQ2, G3IDP5, G3HTF5, G3I4N5, G3HAJ9, G3IAI6, G3I366, G3H485, G3IFE7, G3IHN6, G3H5T7, G3HRL6, G3I0X5, G3HFP8, G3HSA2, G3H2N6, G3HAN5, G3GYD5, G3HHK3, G3I075, G3I2F5, G3H4V0, G3I4H1, G3HQI8, G3HSZ6, G3H8Q6, G3GY22, G3H5W0, G3HQV2, G3HHA3, Q07050, G3H6W0, G3HE31, G3HKV9, G3GRY7, G3HDA0, G3HQ69, P35950, G3I3C7, G3IIU8, G3H121, G3GRS9, G3H3I9, G3HPZ5, G3H9G4, G3HGH4, G3GRA0, G3I277, G3IGZ8, G3IG44, G3HTS3, G3HVP2, G3IJB9, G3HG69, G3I8W2, G3HIU2, O54873, G3HZE8, G3ILX2, G3HFJ1, G3GTH2, G3HIY9, G3IFA9, G3IFT9, G3GX17, G3GW53, G3INX6, G3HXV5, G3HZH3, G3IBK4, G3IP86, G3HLW5, G3H6T8, G3H298, G3GVT2, G3I0X4, G3GXX4, G3HBE9, G3HY75, G3HX67, G3IFV3, G3HQT0, G3I7Q5, G3HYG9, G3I308, G3I9W7, G3HPZ2, G3I8Z5, G3H7Y0, G3HID3, G3HWJ3, G3HWE9, G3I7L9, G3IFL1, G3GW11, G3HP24, G3IJU1, G3H7M3, G3HEV3, G3I1X8, G3HBI9, G3HN30, G3HXY1, G3HU10, G3I7D7, G3HPF1, G3HXW9, P97891, G3H2C9, G3I5L0, G3HN02, G3I2Q7, G3HBY2, G3H1S2, G3HBJ9, G3HWB8, G3I0D8, G3HP03, G3IDU1, G3I2K6, G3HIP2, G3HC25, G3GV81, G3I388, G3HC18, G3IGI9, G3I1P0, G3I0A8, G3HF18, G3H5F7, G3I3W4, G3HRP2, G3HRP3, |
| G3I1J6, P83337, G3H487, G3GZG1, G3I230, G3I9P1, P70102, G3GZB1, G3IJD1, G3H3W4, G3GSZ1, G3GXB1, G3HP08, G3HA24, G3HXU8, G3I1H0, G3H832, G3HV88, G3HQR7, G3HKP7, G3HWC3, G3GWV9, G3IHR6, G3GYY2, G3HJA4, G3H3D3, G3IMX9, G3I9F0, G3I306, G3I1P6, G3HVC8, G3H9C9, G3H953, G3HDE3, G3H1 W2, G3ICY6, G3IE25, G3HFY9, G3I8B9, G3HHZ9, G3H4B6, G3HJS1, G3I0Q0, G3HXU0, G3H2Q4, G3I2Q4, G3I6H3, G3I5H1, G3HE59, G3ILG2, G3H6B2, G3H7F1, G3HQQ9, G3GWC4, G3GY31, G3HLX0, G3HCU8, G3GZ94, G3HWS3, G3GUM9, G3IM40, G3I5W2, G3HYU7, G3I539, G3GR86, G3GTC7, G3HFY1, G3HU24, G3GRM2, G3H066, G3IAE3, G3H0B9, G3H4H6, G3HUJ0, G3H1W1, G3IGS4, G3H8D0, G3HIS0, G3HUL2, G3HAE2, G3HCS4, G3H9F5, G3I3N5, G3HBJ3, G3HHE4, G3IJ29, G3HWS6, G3I3F3, G3I390, G3IE06, G3HN89, Q60446, G3HSA7, G3GTX3, G3I9F1, G3I100, G3H4C1, G3GYJ1, G3IID2, G3IC05, G3HTX6, G3HYI7, G3HQ89, G3I3D5, G3GUC8, G3I837, G3I2B6, G3I8Z3, G3H9D1, G3HTF8, G3HGW5, G3IHH8, G3HQ86, G3GRK8, G3GWX6, G3I2G7, G3H0A7, G3IAW1, G3HMV6, G3HH37, G3H2G5, G3ICC2, G3I970, G3HMM2, G3GVR5, G3HEJ1, G3IH36, G3IP80, G3HFT8, G3I3A8, G3I6I1, G3HC59, G3GVN0, G3HJB6, G3GYX2, G3H1P3, G3IBQ9, G3HEJ4, G3I4R2, G3IJ02, G3H4Z8, G3GZY4, G3GTS6, G3H7T9, G3IB71, G3HID0, G3HN65, G3I7Z8, G3GVW1, G3HGV7, G3HQ74, G3HCL2, G3HP69, G3H229, G3HFG7, G3HIT7, G3GV42, G3H8F2, G3H5M8, G3IHC0, G3HLR7, G3I5Q4, G3HCL3, G3I015, G3IIT5, G3HCT4, G3I3J1, G3IKS9, G3I5T3, G3HEB6, G3I8L4, G3HTM9, G3HFI6, G3HAG8, G3HRW8, G3HRJ1, G3I018, G3I531, G3HJG6, G3H2U4, G3IND2, G3H9Z1, G3HK56, G3I5E0, G3H3W5, G3H256, G3I142, G3I310, G3H2Z7, G3IGW0, G3HJM4, G3IPK9, G3HDD8, G3HBD6, G3HSM8, G3HCI6, G3IBV7, G3HH71, G3HA51, G3GUR0, G3HTE6, G3H0Y9, G3GSF3, Q2MH30, G3GVA8, G3I0R3, G3GRB1, G3HS21, G3HWJ5, G3GRH9, G3H627, G3IJB6, G3ID08, G3GTF3, G3GSH5, G3IHJ3, P49130, G3IL48, G3HV50, G3HLL4, G3GTC9, G3GVZ1, G3GTN1, G3H7Y4, G3HAR2, G3HC17, G3HU02, G3I2H6, G3GUN0, G3HHL3, G3HCV7, G3GZI0, G3H8Z9, G3IHR8, G3H283, G3H9U3, G3HMM0, G3I2M0, G3GV30, G3HNV5, P18708, G3HLX3, G3I873, G3HF56, G3I710, G3H8Y0, G3IF44, G3GU92, G3H902, G3IPV6, G3GS78, G3H125, G3H204, G3GTE4, F7J0L2, G3I0C6, G3HK40, G3I2M6, G3HZU7, G3IH34, G3GWD3, G3HHU4, G3GX38, G3GVY7, G3HV92, G3HKA5, G3GYG0, G3H346, G3HIY6, G3HW44, G3I5V6, G3GYK8, G3GVM9, G3I887, G3H5G6, G3H576, G3HH63, G3H2D6, G3HCG1, G3HQ98, G3HV83, G3H451, G3I7W3, G3H4H4, G3HVX3, G3HV16, G3HV49, G3H3I2, G3IKF4, G3HLS5, G3HS06, G3HI96, G3HCB0, G3IJD6, G3H9I9, G3HB42, G3HS35, G3HDI8, G3IAI5, G3IHH6, G3IN81, G3HTW7, G3HVB8, G3HCX7, G3HI85, G3I4G1, G3I4I9, G3HFW9, G3HR63, G3IG19, G3IHH9, G3H8H7, G3H7V9, G3HHX7, G3H3P5, G3I0C8, G3GRT8, G3HZC7, G3HM98, G3HAN1, G3HSM4, |
| G3IG25, G3I179, G3H9Y7, G3HXR8, G3I2P8, G3H201, G3HT77, G3HBR6, G3HC95, G3HHE3, G3INC8, G3I003, G3GZ89, G3HZY5, G3GVW2, G3HCW3, G3H277, G3H5Z5, G3IFH7, G3HQ88, G3H2J7, G3GYB7, G3GZA9, G3H2U6, G3HET2, G3H571, G3HNP4, G3HVZ2, G3HN66, G3H7T2, G3HAR7, G3HZ17, G3I319, G3II52, G3HEW0, G3HNP6, G3ICW1, G3HF68, G3I2N4, G3ILT7, G3I5N5, G3I0U4, G3H2A5, G3I2X3, G3IDR6, G3HR95, G3HL16, G3I3V3, G3H265, G3H037, G3I1D2, G3GTV9, G3H156, G3HBH4, G3GV64, G3HX83, G3H2H8, G3H470, G3II33, G3GZ81, G3HHC1, G3I6H5, G3IC54, G3H2V3, G3I5L5, G3IGS8, G3HCT1, G3HCL8, G3I2I1, G3HRF0, G3HZG2, G3H5N5, Q9Z1E9, G3GYY4, G3HAT2, G3HHA9, G3GU75, G3I5W9, G3I4G8, G3I1Z7, G3I9B1, G3HXA2, G3GYX1, G3GSR9, G3HS71, G3I198, G3H8T0, G3H9J0, G3IAA6, G3H853, G3H3Z5, G3GXC8, G3HE38, G3HDM0, G3HBG5, G3I6F9, G3I2F9, G3HHX0, G3GX44, G3HUM5, G3HD57, G3HH35, G3HHY9, G3HP80, G3GZQ9, G3GXX3, G3IGN7, G3HMG1, G3IH35, G3HE00, G3H0T9, G3GUG7, G3ILH7, G3HPY5, G3HG36, G3GWF4, G3HG78, G3HCB6, G3GUS2, G3HS40, G3IA32, G3H1H6, G3HIK1, G3GXW9, G3HYE5, G3HJA9, G3GW47, G3GZ54, G3HBZ8, G3HY07, G3HQK5, G3HXT3, G3HIC4, G3HMN4, G3H3S2, G3IH19, G3HRX0, G3I303, G3HF59, G3I3Z5, G3GXU2 |

After the initial data analysis characterization, a list of target HCPs of particular interest was identified based on the above screening. Three subsets of HCP targets were defined as follows: first, all relevant HCPs that could be quantified were identified. These are summarized in Table 1. Next, HCPs were sub-categorized into acHCPs and drHCPs (cf. **Tables 4** and **5**)**.** All HCPs that are either an acHCP, a drHCP, or both following the standards established above are summarized in **Table 2.** Finally, a merged list of those HCPs that qualify as acHCP and as drHCP was defined. This list of targets of high importance is shown in **Table 3.**

To complete the initial classification of HCPs as a basis for subsequently identifying targeted knock-out panels, the relevant HCP knock-out targets from the group of difficult-to-remove HCPs were defined based on the following criterion: the HCPs are present in at least one of the samples shown in the right column in **Fig. 1****,** i.e., the HCP cannot be removed by at least one of the purification strategies used.

With the above information, a complex matrix of single and multiple knock-out strategies was defined and performed as detailed in the following.

### Example 3: Generation and evaluation of single cell clones with single HCP KOs

17 genes (Lpl, Fn1, Prdx1, Bgn, Spp1, Thbs1, Ctsb, Psap, Tnx, Lamb1, Itih5, Nid1, Pxdn1, Lgals3bp, Pcolce, Timp, Hspg2, cf. **Table 3**) identified as abundant core and/or difficult-to-remove HCPs were selected as targets for the generation of CHO DG44 single cell clones with single knock-outs. For all HCP genes, gRNAs targeting early exons were designed using Geneious Prime or Benchling software in combination with the annotated genome of an in-house CHO DG44 suspension cell line. To perform knock-outs, RNP complexes were formed by incubating 7.5 pmol CRIPSR nuclease with 7.5 pmol of the respective target gRNA for 15 min. Subsequently, 2E5 CHO DG44 cells were transfected with the complexes using a Neon transfection system (Thermo), transferred into 1 mL CD DG44 medium and incubated at 36.8 °C and 7.5% CO₂. On day 3 after transfection, single clones were isolated using CellCelector nanowell plates (Sartorius) and a CellCelector system (Sartorius). After an incubation period of 4 days (36.8 °C and 7.5% CO₂), the CellCelector system was used to automatically identify outgrown colonies derived from a single cell and to transfer 96 clones to 384 well plates. Cell growth was monitored with a CellMetric imaging system (Solentim) and clones reaching > 17% confluency were transferred to 96 well plates. Following the isolation of genomic DNA using DNA QuickExtract^{™} DNA Extraction Solution 1.0 (Lucigen), PCR reactions were performed with appropriate primer pairs resulting in 300-500 bp amplicons covering the target regions around the cut sites. The obtained DNA fragments were subjected to Sanger sequencing (Microsynth Seqlab) and analyzed with the open-source software Inference of CRISPR edits (ICE) v3.0 (Synthego) to generate InDel profiles of the edited clones. For each target, 12 clones showing exclusively out-of-frame InDels and thus a complete functional knock-out of the respective HCP were identified and expanded up to shake flask level. Afterwards, the impact of the single knock-outs on the growth and productivity performance of the edited cell clones under bioprocess conditions was evaluated. For that purpose, 1E6 cells of each knock-out clone as well as unedited CHO DG44 for comparison reasons (in triplicates) were transfected with 10 mg of a proprietary expression plasmid coding for a monoclonal antibody and DHFR as a selective marker. In view of the huge number of clones tested, the experiments were conducted in five rounds each, always using CHO DG44 as control. This five-partite experimental set-up is reflected by the dotted lines in **Figure 3A to D.**

2 days post transfection the cells were transferred to a proprietary selective medium, cultivated at 36.8 °C, 7.5% CO₂ and linear shaking at 110 rpm and subcultured every 3-4 days. About 10-14 days after transfection, when increasing cell concentrations and viabilities indicated full recovery from selective pressure, the cultures were used to run fed-batch bioprocesses in 125 mL shake flask: 25 mL proprietary production medium were inoculated with 3E5 cells/mL and cultured at 36.8 °C, 7.5% CO₂ and linear shaking at 110 rpm until the end of the run. Starting on day 3 after inoculation, every day defined amounts of proprietary feed medium A and B as well as glucose were added according to an in-house feeding regime. Viable cell concentration and viability, glucose and lactate concentration as well as antibody titers were measured at defined days of the process using a Vi-Cell cell counter (Beckman Coulter), a Biosen C-Line device (EKF) and an Octet device (Sartorius), respectively. A run was ended once the viability dropped below 70%. As key performance indicators highest viable cell concentration (=peak VCC), process duration, final titer and mean specific productivity were determined for each run and normalized to the respective mean values obtained for the controls (unedited CHO DG44 cells). As summarized in **Figure 3** **A to D,** none of the single knock-outs resulted in a significantly negative impact on growth, process duration, final titer or specific productivity, whereas a positive impact on process duration and final titer was observed in cells containing an Fn1 knock-out.

### Example 4: Generation and evaluation of cell pools with single HCP knock-outs

10 further genes (Pkm, Cspg4, Ldha, Vim, Hspa5, Ppib, Hsp90aa1, Lama5, Fstl1, Aebp1) identified as abundant core and/or difficult-to-remove HCPs were selected as targets for the generation of CHO DG44 cell pools with single knock-outs. For all HCP genes, gRNAs targeting early exons were designed using Geneious Prime or Benchling software in combination with the annotated genome of an in-house CHO DG44 suspension cell line. To perform knock-outs, RNP complexes were formed by incubating 3.75 pmol CRIPSR nuclease with 3.75 pmol of the respective target gRNA for 15 min. Subsequently, 2E5 CHO DG44 cells were transfected with the complexes using a Neon transfection system (Thermo), transferred into 1 mL CD DG44 medium and incubated at 36.8 °C and 7.5% CO₂. 2 days later, the transfected cell pools were transfected a second time with RNP complexes as described above, followed by a third round of transfection after 2 additional days. Following the expansion of the cell pools up to shake flask level, genomic DNA was isolated using DNA QuickExtract^{™} DNA Extraction Solution 1.0 (Lucigen) and PCR reactions were performed with appropriate primer pairs resulting in 300-500 bp amplicons covering the target regions around the cut sites. The obtained DNA fragments were subjected to Sanger sequencing (Microsynth Seqlab) and analyzed with the open-source software Inference of CRISPR edits (ICE) v3.0 (Synthego) to determine the proportion of out-of-frame InDels (and therefore the proportion of functional knock-outs) of the respective HCP in the cell population. Afterwards, the impact of the single knock-outs on the growth and productivity performance of the edited cell pools under bioprocess conditions was evaluated. For that purpose, 1E6 cells of each knock-out pool as well as unedited CHO DG44 for comparison reasons were transfected with 10 mg of a proprietary expression plasmid coding for a monoclonal antibody of interest and DHFR as a selective marker (all transfections were done in triplicates). 2 days post transfection the cells were transferred to a proprietary selective medium, cultivated at 36.8 °C, 7.5% CO₂ and linear shaking at 110 rpm and subcultured every 3-4 days. About 10-14 days after transfection, when increasing cell concentrations and viabilities indicated full recovery from selective pressure, the cultures were used to run fed-batch bioprocesses in 125 mL shake flask: 25 mL proprietary production medium were inoculated with 3E5 cells/mL and cultured at 36.8 °C, 7.5% CO₂ and linear shaking at 110 rpm until the end of the run. Starting on day 3 after inoculation, every day defined amounts of proprietary feed medium A and B as well as glucose were added according to an in-house feeding regime. Viable cell concentration and viability, glucose and lactate concentration as well as antibody titers were measured at defined days of the process using a Vi-Cell cell counter (Beckman Coulter), a Biosen C-Line device (EKF) and an Octet device (Sartorius), respectively. A run was ended once the viability dropped below 70%.

As key performance indicators highest viable cell concentration (=peak VCC), process duration, final titer and mean specific productivity were determined for each run. As summarized in Fig. 4A to D, none of the single knock-outs resulted in a pronounced negative impact on growth, process duration, final titer or specific productivity.

### Example 5: Generation and evaluation of single cell clones with multiple HCP KOs

7 knock-out targets (Bgn, Fn1, Lpl, Nid1, Pcolce, Pxdn, Thbs) were selected based on the data generated in **Example 3** for the generation of CHO DG44 single cell clones with multiple knock-outs. For each of the 7 targets RNP complexes were formed by incubating 1.1 pmol CRIPSR nuclease with 1.1 pmol of the respective target gRNA for 15 min, using the same gRNAs as described in **Example 3.** Subsequently, all 7 RNP reactions were pooled together, 2E5 CHO DG44 cells were transfected with the RNP pool using a Neon transfection system (Thermo), transferred into 1 mL CD DG44 medium and incubated at 36.8 °C and 7.5% CO₂. 2 days later, the transfected cell pool was transfected a second time with pooled RNP complexes as described above, followed by a third round of transfection after 2 additional days. 3 days after the third round of transfection, single clones were isolated using CellCelector nanowell plates (Sartorius) and a CellCelector system (Sartorius). After an incubation period of 4 days (36.8 °C and 7.5% CO₂), the CellCelector system was used to automatically identify outgrown colonies derived from a single cell and to transfer 172 clones to 384 well plates. Cell growth was monitored with a CellMetric imaging system (Solentim) and 158 clones reaching > 17% confluency were transferred to 96 well plates. Following the isolation of genomic DNA using DNA QuickExtract^{™} DNA Extraction Solution 1.0 (Lucigen), PCR reactions were performed with appropriate primer pairs resulting in 300-500 bp amplicons covering the target regions around the cut sites. The obtained DNA fragments were subjected to Sanger sequencing (Microsynth Seqlab) and analyzed with the open-source software Inference of CRISPR edits (ICE) v3.0 (Synthego) to generate InDel profiles of the edited clones. All clones showing exclusively out-of-frame InDels and thus complete functional knock-outs for at least 4 out of the 7 addressed HCP targets were identified and expanded up to shake flask level. The observed knock-out combinations and the number of clones which were found for each of them are summarized in Fig. 5. Afterwards, the impact of the different knock-out combinations on growth and productivity performance of the edited cell clones under bioprocess conditions was evaluated. For that purpose, 1E6 cells of each knock-out clone as well as unedited CHO DG44 for comparison reasons (in quadruplicate) were transfected with 10 mg of a proprietary expression plasmid coding for a monoclonal antibody and DHFR as a selective marker. 2 days post transfection the cells were transferred to a proprietary selective medium, cultivated at 36.8 °C, 7.5% CO₂ and linear shaking at 110 rpm and subcultured every 3-4 days. About 10-14 days after transfection, when increasing cell concentrations and viabilities indicated full recovery from selective pressure, the cultures were used to run fed-batch bioprocesses in 125 mL shake flask: 25 mL proprietary production medium were inoculated with 3E5 cells/mL and cultured at 36.8 °C, 7.5% CO₂ and linear shaking at 110 rpm until the end of the run. Starting on day 3 after inoculation, every day defined amounts of proprietary feed medium A and B as well as glucose were added according to an in-house feeding regime. Viable cell concentration and viability, glucose and lactate concentration as well as antibody titers were measured at defined days of the process using a Vi-Cell cell counter (Beckman Coulter), a Biosen C-Line device (EKF) and an Octet device (Sartorius), respectively. A run was ended once the viability dropped below 70%. As key performance indicators highest viable cell concentration (=peak VCC), process duration, final titer and mean specific productivity were determined for each.

As summarized in **Fig. 6****,** for none of the analyzed HCP knock-out combinations a pronounced negative impact on growth, process duration, final titer or specific productivity compared to unedited CHO DG44 cells was observed. This confirms that the targets represent suitable targets for multiple KOs for reducing the overall HCP load, whilst at the same time maintaining the cellular integrity.

### Example 6: Nuclease Testing

Having confirmed that multiple HCP knock-outs can be inserted into a CHO-target cell of interest simultaneously without a negative impact on the overall viability and production capability of the target cell, several nucleases were chosen for testing to define the optimum set-up for a multiplex knock-out experiment. To this end, several multiple targets (at least 5 at the same time) as shown in Table 1 were selected. Further, suitable nucleases were provided and the guide RNAs were adapted accordingly to fit the needs of the nuclease of interest and to recognize the respective target sequences, respectively.

It could be shown that certain nucleases perform superior in multiplexing knock-out experiments. Generally, it can be preferable to knock-out more than one HCP target in once experiment to reduce the overall process time needed, the decrease the cellular stress on the target cell and to better control and check the knock-out efficiency and effectiveness.

As it could be demonstrated that several nucleases are suitable for creating multiple desired knock-outs simultaneously, further experiments are currently ongoing to defined alternative strategies.

### Example 7: Further Compositions and Equipment

### Media, Compositions, Kits and Chemicals

For CHO cell cultivation, commercially available media from Thermo Fisher Scientific and Sartorius Stedim Cellca were used.

For further material, 125mL shake flasks of Corning, 24-well Nanowell plates of ALS, 384-well flat clear bottom black microplates of Corning, 96-well PCR plate, non-skirted Brand CASY cups of OMNI Life Science, CASY ton of OMNI Life Science, Enhanced Qsol Cartridge Kit for iQue^{®} screener of Sartorius, iQue^{®} Marker (B/Yellow) of Sartorius, iQue^{®} Validation Beads of Sartorius, NEON^{™} Transfection System 10 µl/100 µl kit of Thermo Fisher Scientific, NucleoSpin^{®} miRNA of Macherey-Nagel, Nunc cell culture platesof Thermo Fisher Scientific, Phusion^{®} High-Fidelity DNA Polymerase of New England Biolabs, pmaxGFP^{®} Vector of Lonza, and QuickExtract DNA Extraction Solution of Lucigen were usually used, but alternative materials can be used as well. For devices and equipment, the following were used, but alternative equipment as available to the skilled person can of course be used as well: Cell counter of Beckman Coulter, Cell counter of Roche, Cell imager of Solentim, CO2 incubator of Thermo Fisher Scientific, Flow cytometer of Sartorius, Glucose/Lactate Analyzer of EKF-diagnostics, Protein Quantification System of Sartorius, Real-Time PCR device of Thermo Fisher Scientific, Single cell cloning device of Automated Lab Solutions, and a Transfection device of Thermo Fisher Scientific.

### Example 8: sgRNAs

To knock out the desired gene targets, sgRNAs were designed targeting an early exon present in all available transcript variants utilizing the bioinformatics tool Geneious Prime 11.0.11 (Biomatters, Auckland, New Zealand) or the cloud-based CRISPR Guide RNA Design Software (https://www.benchling.com). The sgRNAs were then ordered and synthesized by different manufacturers, depending on the nuclease of interest the sgRNA was designed for. To generate frameshift mutations in the desired HCP genes, usually at least three sgRNAs for each target were evaluated for their KO-efficiency regarding their ability to generate desirable InDels leading to a functional KO. Desirable InDels are defined as InDels which are not multiples of three, show a large prevalence (>30%) in the heterogenous sequence pool and lead to early stop-codons in translated protein sequences. In the first KO-round, the CHO DG44 host cell pool was transfected with sgRNAs for each target gene as shown in Tables 1 to 3 above. R-buffer transfected cells served as negative controls and pMaxGFP as positive control for the determination of transfection efficiency and assessment of the viability of the transfection setup of devices and reagents. Transfection efficiency was determined in two separate LPs 24 h after transfection in three technical replicates, gating and efficiencies (data not shown here). The total mean of transfection efficiency was measured to be 96.9% (data not shown). Genomic DNA was extracted 48 h after transfection. Via Sanger sequencing, InDel events (i.e. insertions or deletions) at cut sites of the respective site-directed endonuclease were assessed in DNA fragments of pools and clones previously amplified via PCR. The chromatograms from sequencing reactions were viewed using Geneious Prime 11.0.11 (Biomatters, Auckland, New Zealand). Also, a quality score was assigned to each sequencing reaction corresponding to the percentage of untrimmed bases that are high quality, where high quality is defined as a function of the percentage of ambiguities present in the sanger traces. The results obtained via Sanger sequencing could e.g. be used for assessing specific editing efficiencies and InDel profiles. Selected gRNAs used (only crRNA, 5' to 3' w/o PAM) are shown with SEQ ID NOs: 129 to 153.

Wild type CHO cells and all derived clones were handled under sterile conditions at all times. CHO cell cultivation was always performed at 36.8 °C, under 7.5% CO₂ atmosphere while shaking at 110 rpm. Only cultures in plates (i.e. nanowell plates, 384-well plates, 96-well plates, 24-well plates, and 12-well plates) were incubated without shaking. Culture parameters like the viable cell concentration (VCC), total cell concentration (TCC), viability and peak diameter were determined using the CASY cell counter.From the pools transfected with a site-directed endonuclease of interest, stable single-cell clones were generated in order to be assessed in fed-batch mode.

Single-cell cloning was usually performed 3 days after transfection with the respective site-directed endonuclease.

After genetic screening, knock-out clones (KO-clones) were resuspended and diluted and clones were further incubated and cell growth was consistently monitored by measuring confluency.

KO-clones transfected with the respective expression vector (as described above) were expanded. Once good cell growth (about 8 to 10 * 10⁵ cells/mL after days) and a good cell viability of > 90% could be restored after the transfection procedure, a fed-batch bioprocess was performed for evaluation of culture performance of the respective KO-clones.

Antibody titers of samples obtained from fed-batch cultures were determined using Octet^{®} QKe (Sartorius) system according to the manufacturer's instructions. Titers were determined by thawing (RT) supernatant samples collected during fed-batch cultivation. The results were then analysed using the Forte Bio Data analysis 9.0 software (Sartorius Lab Instruments GmbH & Co. KG, Goettingen, Germany).

To screen HCPs present in supernatants from harvested samples, amino acid analysis and peptide fragment matching against protein databases to designate present HCPs with UniProt Identifiers through external liquid chromatography (LC-MS) (Alphalyse A/S, 5230 Odense M, Denmark) was performed. Additionally, for HCP profiling screens, SWATH LC-MS was used (cf. Krasny et al., Journal of Proteomics Volume 189, 2018).

The relative abundance of each HCP could then be calculated utilizing the production titer provided by Sartorius. Further data about the cellular localization, amino acid sequence, gene ID and gene structure as well as functions of each HCP was acquired from the UniProt Database. The genetic sequences of all proteins were acquired through NCBI and verified.

## Claims

1. A CHO cell with a modified host cell protein (HCP) profile **characterized by** a reduced load of at least one endogenous protein being a difficult-to-remove HCP (drHCP) and/or an abundant core HCP (acHCP), wherein said CHO cell comprises:
at least one modification in at least one endogenous coding sequence coding for at least one drHCP and/or acHCP selected from the group summarized in Table 1 or any homologue, orthologue, or paralogue thereof.

2. A CHO cell with a modified host cell protein (HCP) profile **characterized by** a reduced load of at least one endogenous protein being a difficult-to-remove HCP (drHCP) and/or an abundant core HCP (acHCP), wherein said CHO cell comprises:
two or more modifications in two or more coding sequences coding for two or more drHCPs and/or acHCPs defined / shown in Table 2, 3, 4 or 5, or any homologue, orthologue, or paralogue thereof.

3. The CHO cell according to claim 1 or 2, wherein at least one, or the two or more modification(s) affect(s) a drHCP, preferably wherein the at least one, or the two or more modification(s) affect(s) one, two, or more protein(s) being both classified as a drHCP and as an acHCP, or wherein said at least one endogenous protein has an amino acid sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 1, 2, 4, 5, 6, 7, 8, 9, 11, 12, 13, 127, 14, 16, 17, 19, 20, 21, 22, 23, 24, 27, 31, 34, 35, 41, 49, and 55, respectively, preferably wherein said at least one endogenous protein has an amino acid sequence corresponding to SEQ ID NO: 2, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 2.

4. The CHO cell according to any of the previous claims, wherein said at least one modification leads to the reduced transcription and/or reduced functional expression of said endogenous protein(s) thereby lowering the total content of drHCPs and/or acHCPs.

5. The CHO cell according to any of the previous claims, wherein said CHO comprises at least one recombinant gene encoding at least one recombinant protein of interest and/or encoding at least one recombinant RNA molecule of interest.

6. The CHO cell according to claim 5, wherein said at least one recombinant protein of interest is a therapeutic molecule.

7. The CHO cell according to any of the previous claims, wherein said at least one modification is selected from the group consisting of at least one insertion, at least one deletions, and at least one substitution, including a base edit, or any combination thereof,
preferably wherein said at least one modification is present in exon 1 of the respective endogenous coding sequence,
and/or
wherein said at least one modification in said coding sequence is a frame-shift mutation or a point mutation, the point mutation resulting in a stop codon.

8. The CHO cell according to any of the previous claims, wherein said at least one modification in said endogenous coding sequence(s) allows for an optimized downstream processing and/or guarantees a reduced load of total HCPs,
(i) wherein the CHO cell comprises at least one recombinant non-endogenous gene encoding at least one recombinant protein or RNA of interest and wherein said optimized downstream processing is **characterized by** a reduced load of total HCPs in the recombinant protein or RNA of product of interest, wherein the total load of HCPs is reduced by at least 0.1%, at least 0.2%, at least 0.3%, at least 0.4%, at least 0.5%, at least 0.6%, at least 0.7%, at least 0.8%, at least 0.9%, preferably at least 1%, at least 1.25%, at least 1.5%, at least 1.75%, at least 2%, at least 2.25%, at least 2.5%, at least 2.75%, at least 3%, at least 3.25%, at least 3.5%, at least 3.75%, at least 4%, at least 4.5%, more preferably at least 5%, at least 5.5, at least 6%, at least 6.5%, at least 7%, at least 7.5%, at least 8%, and even more preferably at least 9%, at least 10%, at least 15%, at least 20%, or at least 25% as determined by measuring the relative concentration of total HCPs in ng/mL per sample obtained in a modified CHO cell in comparison to a reference sample obtained from a CHO wild-type cell,
and/or
(ii) wherein said at least one modification in said endogenous coding sequence(s) leads to an improved upstream processing performance,
wherein said improved upstream processing performance is **characterized by** an increased concentration of viable cells and/or an increased specific productivity and/or an increased final titer and/or an increased process duration in comparison to a wild-type cell not carrying the at least one modification in its genome.

9. The CHO cell according to any of the previous claims, wherein all alleles of said at least one endogenous coding sequence comprise at least one or more of said modification(s).

10. The CHO cell according to any of the previous claims,
wherein said at least one endogenous protein has an amino acid sequence corresponding to any of the amino acid sequences according to SEQ ID NOs: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, and 128 or corresponding to an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to any one of the sequences according to SEQ ID NOs: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, and 128.

11. The CHO cell according to any of the previous claims, wherein the CHO cell comprises at least one modification in at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty or more different HCP encoding sequences, wherein each HCP is preferably selected from an acHCP and/or a drHCP.

12. A method for the production of at least one modified CHO cell according to any of the claims 1 to 11 comprising the steps:
(i) providing at least one CHO cell comprising at least one endogenous target nucleic acid segment;
(ii) providing at least one genome or transcriptome editing agent comprising at least one RNAi agent, or at least one site-directed endonuclease, preferably being selected from a meganuclease, a ZFN, a TALEN, a CRISPR-nuclease, or a nickase or nuclease-dead variant therefrom, or a nucleic acid molecule encoding the same, and optionally in case of a CRISPR-nuclease: providing at least one suitable, functional guide RNA molecule, or a nucleic acid molecule encoding the same;
(iii) introducing into said at least one CHO cell the at least one genome editing agent of step (ii);
(iv) obtaining at least one modified CHO cell comprising at least one modification in said at least one endogenous target nucleic acid segment according to step (i);
(v) optionally: selecting a further target nucleic acid segment and repeating steps (i) to (iv) at least one time to obtain at least one modified CHO cell comprising at least one further modification in a further endogenous target nucleic acid segment.

13. A method for the production of at least one recombinant molecule, preferably at least one recombinant protein, of interest comprising the steps:
(a) providing at least one modified CHO cell according to any of the claims 1 to 11, wherein the at least one modified CHO cell comprises at least one recombinant gene encoding at least one recombinant protein, DNA or RNA of interest;
(b) culturing said at least one target cell in a culture medium such that at least one recombinant molecule of interest is transcribed and/or translated;
(c) harvesting said at least one recombinant molecule of interest;
(d) purifying and/ or decontaminating said at least one recombinant molecule, preferably the at least one recombinant protein of interest.

14. The use of at least one modified CHO cell according to any of the claims 1 to 11 for the production of at least one pharmaceutical drug.

15. A kit comprising
(a) at least one modified CHO cell according to any of claims 1 to 11, the CHO cell or the kit comprising:
(b.i) at least one nucleic acid molecule suitable for expressing at least one recombinant molecule of interest, preferably at least one recombinant protein of interest; optionally means for introducing the same into the CHO cell genome, or alternatively
(b.ii) at least one nucleic acid molecule encoding at least one recombinant molecule of interest, preferably at least one recombinant protein of interest; optionally means for introducing the same into the CHO cell genome; or alternatively
(b.iii) at least one nucleic acid molecule suitable for transcribing at least one recombinant RNA molecule of interest; optionally means for introducing the same into the CHO cell genome; or alternatively
(b.iv) at least one nucleic acid molecule encoding at least one recombinant RNA molecule of interest; optionally means for introducing the same into the CHO cell genome; and optionally
(c) culture medium suitable for the at least one modified CHO cell according to step (a)
(i) to replicate the at least one nucleic acid molecule according to any of the steps (b.i), (b.ii), (b.iii), and (b.iv) endogenously or exogenously; and
(ii.a) to express the at least one recombinant protein of interest according to step (b.i) and/or (b.ii), and/or to transcribe the at least one recombinant RNA molecule of interest according to step (b.iii) and/or (b.iv).
